# FASCICULE DE BREVET EUROPEEN

(11) **EP 3 806 913 B1**
(45) Date de publication et mention de la délivrance du brevet: **18.05.2022**
(21) Numéro de dépôt: 19790602.7
(22) Date de dépôt: 18.06.2019
(51) Int. Cl.: A61L 9/12, A01M 1/20, A01M 29/12, A01M 7/00

(54) **APPAREIL POUR DISPERSER DANS L'AIR UNE VAPEUR DE SUBSTANCE LIQUIDE**
APPARAT ZUM DISPERGIEREN EINES DAMPFES EINER FLÜSSIGEN SUBSTANZ IN DER LUFT
APPARATUS FOR DISPERSING IN THE AIR A VAPOUR OF A LIQUID SUBSTANCE

(30) Priorité: 18.06.2018 FR 1855310; 02.11.2018 FR 1860145
(43) Date de publication de la demande: 21.04.2021
(73) Titulaire: CAELIMP, 31670 Labege (FR)
(72) Inventeur: PEREZ, Yoann, 65410 Sarrancolin (FR); RIVIERE, Philippe, 31500 Toulouse (FR); PICHON, Philippe, 31800 VILLENEUVE-DE-RIVIERE (FR)
(74) Mandataire: Loyer & Abello
(86) Numéro de dépôt international: PCT/FR2019/051490
(87) Numéro de publication internationale: WO 2019/243734

(56) Documents cités:
- EP-A1- 1 846 044
- WO-A1-03/092750
- US-A- 2 140 516
- US-A1- 2011 049 259
- US-A1- 2018 000 977

## Description

La présente invention a pour objet un appareil pour disperser, à l'état vapeur dans un flux d'air, une substance, qui est sous forme liquide à température ambiante. La difficulté pour obtenir une telle dispersion est généralement de faire en sorte que la dispersion soit sensiblement uniforme dans le temps et dans le flux d'air et que la concentration de la substance par rapport audit flux puisse être très faible. En effet, le problème se pose, notamment, quand on veut diffuser, dans une zone fermée de grand volume, un parfum, ou, dans une zone ouverte, telle qu'un champ cultivé, un produit phytosanitaire utile à la culture dans ledit champ ; le même problème se pose aussi quand on veut disperser en plein champ un produit semiochimique tel qu'une phéromone susceptible d'agir pour contrôler les insectes nuisibles à la culture faite dans ledit champ.

On a déjà proposé des systèmes permettant d'obtenir une libération lente et continue de substances liquides actives ; ces substances sont généralement des liquides utilisés tels quels ou absorbés sur différents supports ; le principe actif peut notamment se trouver dans des matrices polymères non poreuses, dans des microcapsules à enveloppe polymérique (brevet US 3577515), dans des gels (brevet US 2800457) ou encore dans des fibres creuses (brevet US 4017030). Les principes actifs se répartissent dans l'air ambiant par diffusion passive (simple évaporation atmosphérique). Malheureusement, la cinétique de libération des principes actifs est influencée par les facteurs ambiants, ce qui ne permet pas d'agir efficacement pour réguler la vitesse de libération des principes actifs.

En outre, lorsque la substance liquide est constituée de plusieurs composés, qui n'ont pas les mêmes températures d'évaporation, la substance se modifie au fur et à mesure de l'évaporation, de sorte que la durée d'activité de ce type de dispositif est incertaine.

On a déjà proposé également des systèmes pour contrôler la diffusion d'une substance volatile en assurant par un système de chauffage une évaporation maitrisée du principe actif ; on a même proposé des systèmes à mèche dans lesquels on ne chauffe que la solution liquide se trouvant dans la mèche où se produit l'évaporation (voir notamment les brevets EP 1579762 et 2481308) ou dans un textile (brevet français 2722368).

On a aussi proposé de chauffer un réservoir de la substance liquide à vaporiser. Mais on a alors constaté qu'un problème se posait généralement pour obtenir par chauffage une diffusion de composition constante (demande de brevet US 2007/0257016). Dans tous ces systèmes comportant uniquement un chauffage comme contrôleur de l'évaporation, il n'est donc pas possible de déterminer, précisément pour un moment donné, le débit de vapeur de la substance, non plus que la durée pendant laquelle ce débit est distribué, sans connaître les autres variables relatives à l'évaporation d'une substance (dont, notamment, le flux d'air à l'interface, les propriétés physico-chimiques de la substance, l'interaction entre la substance et les surfaces environnantes).

En outre, la diffusion de la substance en plusieurs points d'un espace traité ne peut se faire que par plusieurs dispositifs, placés chacun en l'un des points de diffusion utilisés, ce qui ne garantit pas l'égalité entre les distributions faites dans les différentes zones traitées selon la différence des variables exogènes aux systèmes et spécifiques à chaque endroit. Le coût des systèmes antérieurement connus n'est donc pas grevé seulement par la consommation de la substance dispersée, mais également souvent par l'obligation, pour obtenir une efficacité acceptable, de mettre en œuvre un nombre important de dispositifs par hectare de surfaces à traiter ; l'installation de ces dispositifs est coûteuse, mais également leur surveillance pour éviter que des incidents de fonctionnement ne génèrent des pollutions environnementales localisées.

Ainsi un but de l'invention est de fournir un appareil ne présentant pas certains des inconvénients précédemment mentionnés.

Le document US 2018/000977 A1 divulgue un appareil conforme au préambule de la revendication 1. D'autres dispositifs ou appareils pour disperser à l'état vapeur une substance liquide sont connus par les documents WO 03/092750 A1, EP 1 846 044 A1, US 2,140,516 A ou US 2011/049259 A1. Certains aspects de l'invention partent de l'idée de proposer un appareil de dispersion dont la consommation d'énergie soit faible pour assurer une grande durée d'autonomie.

Certains aspects de l'invention partent de l'idée de proposer un appareil de dispersion qui régule un débit de substance distribuée par un simple contrôle de température de l'organe diffuseur.

Certains aspects de l'invention partent de l'idée de proposer un appareil de dispersion adapté particulièrement à distribuer une substance de grande valeur avec une précision élevée sans pertes de substance.

A cet effet, la présente invention fournit un appareil pour disperser dans l'air, à l'état de vapeur, une substance à l'état liquide à température ambiante et contenue dans un récipient de stockage. L'appareil comporte :
- un système d'aération comportant une canalisation débouchant à l'air libre et configuré pour permettre le passage d'un débit d'air dans la canalisation ;
- au moins un organe distributeur destiné à être alimenté en une substance liquide par le récipient de stockage, l'organe distributeur comportant des micro-canalisations formant une sortie agencée dans la canalisation afin d'y constituer une zone d'évaporation de la substance,
- au moins un récipient de stockage contenant la substance et relié à l'organe distributeur, le récipient présentant un orifice de vidange relié à l'organe distributeur, et
- un organe de chauffage agencé sur ou dans l'organe distributeur de manière à contrôler un écoulement de la substance à travers l'organe distributeur. L'orifice de vidange est orienté vers le bas lorsque l'appareil est dans une position d'utilisation, l'organe distributeur étant situé en dessous de l'orifice de vidange dans la position d'utilisation, et la substance présente une viscosité variable en fonction de la température, ladite viscosité étant telle que la substance ne peut pas s'écouler à travers les micro-canalisations de l'organe distributeur à une température ambiante inférieure à une première température, l'organe de chauffage étant configuré pour chauffer l'organe distributeur jusqu'à une deuxième température supérieure à la première température de sorte qu'un écoulement de la substance à travers les micro-canalisations de l'organe distributeur se produit par capillarité.

Dans le cadre d'une substance onéreuse, par exemple la substance comprend une phéromone, se présentant sous forme liquide à température ambiante, il faut éviter d'en gaspiller. Ainsi dans ce cas figure, on désire amener une quantité de liquide suffisamment faible pour que l'écoulement s'effectue sans formation de goutte, mais néanmoins, suffisamment grande pour que la zone d'évaporation reste mouillée en permanence malgré le débit d'air envoyé par le système d'aération.

Ce phénomène physique est gouverné à froid par la loi de Jurin et à chaud par la loi de Darcy.

La loi de Darcy s'énonce ainsi Q = KA (ΔH)/L, où Q est le débit volumique, K est la conductivité hydraulique, A est la surface de la section étudiée, ΔH est la différence des hauteurs piézométriques en amont et en aval de l'échantillon et L est la longueur de l'échantillon. La conductivité hydraulique se calcule avec la formule K = kρg/µ, où k est la perméabilité intrinsèque du milieu poreux, ρ est la masse volumique du fluide, g est l'accélération de pesanteur et µ est la viscosité du fluide.

La loi de Jurin correspond à la formule h = (2γcos(θ))/(rρg), où h est la hauteur du liquide, y est la tension superficielle du liquide, θ est l'angle de contact entre le liquide et la paroi des micro-canalisations, ρ est la masse volumique du liquide, r est le rayon des micro-canalisations, et g est la constante de la gravité.

On désire se placer dans les conditions où à froid, K est trop faible pour qu'il y ait un écoulement c'est-à-dire qu'on se trouve dans une situation dite « capillaire », et où à chaud l'écoulement est suffisant pour qu'il y ait étalement en surface et que le liquide adhère à la surface. La couche de liquide adhérant à la surface modifie ΔH et on a un débit figé du fait que K a atteint une valeur maximale.

Les deux paramètres les plus importants sont donc la viscosité du fluide et la température.

Dans un exemple de réalisation, cosθ est positif c'est-à-dire que la substance est mouillante sur l'organe distributeur, par exemple en céramique, la masse volumique du liquide est comprise entre 0,6 et 1g/cm³, et le rayon des micro-canalisations est compris en 5 nm et 1 µm.

A froid, la surface de liquide évaporable est donc très faible : somme des micro-canalisations, liquide en retrait et froid (donc dépendance de la volatilité du liquide). Pour les phéromones, l'évaporation à froid est nulle.

La baisse de la viscosité dynamique de la substance avec la chaleur fournie par l'organe de de chauffage permet au fluide de circuler au sein de l'organe distributeur selon la loi de Darcy puis de s'étaler en surface dudit organe distributeur. Sans apport de chaleur, la circulation est figée car la somme des adhérences au sein de l'organe distributeur suit la loi de Jurin. En d'autres termes, l'écoulement est permis à travers l'organe distributeur à chaud mais stoppé à température ambiante par la force d'adhérence entre le fluide et la surface de l'organe distributeur.

Lors de l'écoulement, il faut plus d'énergie pour former une goutte qui se détachera que pour maintenir la solution au sein de l'organe distributeur et du récipient de stockage. Cela tient à deux conditions :
1. la viscosité dynamique de la substance ne doit pas être trop faible dans la plage de température pouvant être atteinte à l'aide de l'organe de chauffage, et
2. le liquide en sortie de réservoir doit être en équilibre avec la pression atmosphérique; ce qui peut être mis en œuvre de plusieurs manières. Par exemple, la partie dépourvue de liquide dans le récipient de stockage est en dépression. Alternativement, un système de gestion de pression de la partie dépourvue de liquide du récipient assure cet équilibre.

Dans le présent texte de demande de brevet, on appellera « micro-canalisation » une canalisation, dont la section droite a une aire comprise entre 10⁻⁴ et 10⁶ µm². Selon un mode de réalisation, l'organe distributeur comporte un corps poreux comprenant des pores, lesdits pores constituant au moins une partie des micro-canalisations de l'organe distributeur.

Selon un mode de réalisation, les pores présentent un diamètre compris entre 0.01 et 10 µm.

Selon un mode de réalisation, le corps poreux présente une forme de cylindre. Selon un mode de réalisation, l'alimentation en substance est reçue dans un évidement.

Selon un mode de réalisation l'évidement est aveugle et prévu parallèlement à l'axe du corps poreux.

Selon un mode de réalisation, le corps poreux comprend un ergot agencé sur une partie supérieure dudit corps poreux et s'étendant selon un axe longitudinal du corps poreux et configuré pour recevoir la substance.

Selon un mode de réalisation, l'organe distributeur comporte une membrane périphérique agencée autour du corps poreux et percée de trous constituant des micro-canalisations.

Selon un mode de réalisation, le corps poreux présente une porosité dans une partie intérieure du corps poreux plus faible qu'une porosité dans une partie extérieure du corps poreux entourant la partie intérieure. Cela permet de contrôler le débit d'écoulement dans le corps poreux avec la faible porosité et augmenter les échanges avec l'air avec la forte porosité de surface.

Selon un mode de réalisation, le corps poreux comporte une mèche en bois, en textile, en céramique ou en polymère.

Selon un mode de réalisation, l'organe de chauffage est placé directement sur une surface du corps poreux.

Selon un mode de réalisation, le corps poreux présente au moins un évidement logeant au moins une partie de l'organe de chauffage.

Selon un mode de réalisation, l'organe distributeur comprend une aiguille creuse configurée pour percer un opercule du récipient de stockage et/ou pour déplacer une membrane faisant clapet du récipient de stockage et amener la substance contenue dans le récipient de stockage jusqu'à la surface d'évaporation.

Selon un mode de réalisation, l'aiguille est disposée à une des extrémités du corps poreux. Une telle aiguille peut aussi être employée en combinaison avec un bouchon perforable «auto-cicatrisant» logé dans l'entrée du récipient de stockage, c'est-à-dire une masse de matière élastique qui referme élastiquement la perforation réalisée par l'aiguille, de sorte qu'aucun écoulement n'a lieu après le retrait de celle-ci.

Selon un mode de réalisation, un trajet à partir du récipient de stockage jusqu'à une sortie des micro-canalisations dans la zone d'évaporation ne constitue une micro-canalisation que sur une fraction d'une longueur du trajet.

Selon un mode de réalisation, les micro-canalisations présentent une section comprise entre 10⁻⁴ µm² et 10⁶ µm², de préférence entre 0,1 µm² et 10³ µm². Selon un mode de réalisation le rapport de la section interne de la canalisation du système d'aération à une section droite externe de la zone d'évaporation, est compris entre 1,2 et 625.

Selon un mode de réalisation, l'appareil comporte en outre un organe de fixation orientable en direction et/ou en inclinaison par rapport à la canalisation du système d'aération, afin d'orienter la canalisation par rapport au sol lorsque l'organe de fixation est fixé à un support.

Selon un mode de réalisation, le système d'aération comporte au moins un ventilateur mis en place dans une partie de la canalisation.

Selon un mode de réalisation, le système d'aération comporte au moins un ventilateur mis en place dan la partie de la canalisation, qui est à l'opposé de son débouché à l'air libre.

Selon un mode de réalisation, le système d'aération comporte des ouvertures pratiquées dans une paroi d'extrémité de la canalisation et des obturateurs réglables équipant lesdites ouvertures pour permettre de régler une section de passage des ouvertures.

Selon un mode de réalisation, l'appareil comprend un organe régulateur d'un débit d'air dans la canalisation, configuré pour contrôler le ventilateur et/ou les obturateurs afin de réguler un débit d'air dans la canalisation.

Selon un mode de réalisation, le débit d'air du système d'aération de l'appareil selon l'invention, est associé à un organe régulateur susceptible de contrôler la turbulence du flux d'air au niveau de la zone d'évaporation ; l'organe régulateur peut être piloté par au moins un capteur de température repérant la température du flux d'air et/ou celle du corps poreux ou par au moins un capteur de vitesse repérant la vitesse du flux d'air.

Selon un mode de réalisation l'organe régulateur est configuré pour émettre un signal agissant sur la vitesse de rotation du ventilateur générant le débit d'air dans le système d'aération et/ou un signal agissant sur les obturateurs réglables.

Selon un mode de réalisation le débit d'air du système d'aération est compris entre 0,2 et 60 m³/h.

Selon un mode de réalisation la canalisation est équipée d'un capteur de vitesse et de température du flux d'air.

Dans un mode de réalisation, la tubulure est équipée d'un capteur de vitesse et de température du flux d'air ; le contrôle de turbulence de l'air, où se disperse la substance S, est assuré grâce à au moins un capteur de température repérant la température du flux d'air et/ou celle du corps poreux.

Selon un mode de réalisation un contrôle de turbulence de l'air, où se disperse la substance, est assuré grâce à au moins un capteur de température mesurant la température de l'organe distributeur et/ou la température du flux d'air.

Selon un mode de réalisation, l'appareil comprend en outre un dispositif de commande configuré pour commander l'organe de chauffage en fonction d'une température de consigne dans l'organe distributeur.

Selon un mode de réalisation, l'organe de chauffage comprend au moins une carte électronique et au moins une résistance électrique alimentée électriquement par la carte électronique. La résistance électrique peut être disposée sur ladite carte électronique, ou déportée de celle-ci.

Selon un mode de réalisation, le dispositif de commande est agencé sur la carte électronique.

Selon un mode de réalisation, l'organe distributeur est équipé d'un capteur de température, par exemple au niveau d'une extrémité libre.

Selon un mode de réalisation, la température de consigne est définie en fonction de la substance.

Selon un mode de réalisation, le dispositif de commande est relié à un détecteur configuré pour détecter un marquage au niveau du récipient de stockage indicatif de la substance contenue dans le récipient et le dispositif de commande détermine en fonction dudit marquage, au moins un paramètre de fonctionnement du dispositif parmi la température de consigne, un débit d'air et des indications temporelles définissant un cycle arrêt/marche. De telles indications temporelles incluent par exemple des dates de début de cycle, dates de fin de cycle, durées de cycle, durée inter-cycles, etc.

Selon un mode de réalisation, le dispositif de commande comporte une mémoire stockant une table de valeurs associant des substances à des températures de consigne.

Selon un mode de réalisation, l'appareil comporte en outre un module de communication pour assurer une communication filaire ou non filaire avec un serveur de données, afin de modifier la table de valeurs.

Selon un mode de réalisation, la substance à la deuxième température se répand dans l'état liquide sur une surface de l'organe distributeur située dans le système d'aération.

Selon un mode de réalisation, l'organe de chauffage est configuré pour réguler un débit de la substance à travers l'organe distributeur en modifiant une viscosité de la substance sans vaporiser la substance.

Selon un mode de réalisation, la deuxième température est choisie de manière que l'écoulement de la substance s'effectue à un débit suffisamment faible pour éviter la formation de gouttes se détachant de l'organe distributeur et suffisamment grand pour que la zone d'évaporation reste mouillée en permanence malgré le débit d'air envoyé par le système d'aération.

Hors utilisation dans l'appareil, c'est -à-dire avant que le récipient ne soit mis en connexion avec l'organe distributeur ou après avoir été déconnecté de l'organe distributeur, un tel récipient de stockage peut être muni d'un bouchon agencé sur l'orifice de vidange.

Selon un mode de réalisation, le récipient de stockage ne présente pas d'autre ouverture que l'orifice de vidange, ledit récipient de stockage contenant, outre la substance liquide, une phase gazeuse occupant au moins 20% du volume du récipient de stockage.

Selon un mode de réalisation, le récipient de stockage comporte un réservoir extérieur et un réservoir intérieur logé dans le réservoir extérieur, le réservoir intérieur étant en liaison avec l'organe distributeur à travers l'orifice de vidange et présentant un évent relié à l'atmosphère à une extrémité opposée à l'orifice de vidange, un orifice de communication entre le réservoir extérieur et le réservoir intérieur étant agencé à proximité de l'orifice de vidange, le réservoir extérieur ne présentant pas d'autre ouverture que l'orifice de communication.

Selon un mode de réalisation, le récipient de stockage est monté de manière amovible dans l'appareil et configuré de manière à pouvoir être retiré de l'appareil sans perte de substance.

Selon un mode de réalisation, le récipient de stockage est monté dans l'appareil par vissage ou encliquetage.

Selon un mode de réalisation, l'organe distributeur présente une première surface tournée vers le récipient de stockage et munie d'un joint d'étanchéité assurant une liaison étanche entre l'organe distributeur et le récipient de stockage, et une deuxième surface agencée dans le système d'aération.

Selon un mode de réalisation, le récipient de stockage comprend un joint d'étanchéité agencé autour de l'orifice de vidange, de manière à assurer une liaison étanche entre le récipient de stockage et l'organe distributeur.

Selon un mode de réalisation, le récipient de stockage comprend un organe de rétention alvéolaire agencé dans le récipient de manière adjacente à l'orifice de vidange pour limiter un écoulement de la substance.

Selon un mode de réalisation l'organe de chauffage et le récipient de stockage sont disposés de part et d'autre de l'organe distributeur.

Selon un mode de réalisation l'organe de rétention alvéolaire comprend un matériau choisi parmi un feutre, par exemple feutre en laine, et une mousse de mélamine.

Selon un mode de réalisation, une liaison entre un récipient de stockage et son organe distributeur associé est assurée au moyen d'une tuyauterie équipée d'une électrovanne d'arrêt à la sortie du récipient.

Selon un mode de réalisation, un moyen régulateur de distribution est inséré entre le récipient de stockage de la substance et l'organe distributeur .

Selon un mode de réalisation, le moyen régulateur de distribution est une vanne à ouverture réglable.

Selon un mode de réalisation, la vanne n'a que deux positions de réglage, à savoir, ouverture ou fermeture.

Selon un mode de réalisation, le moyen régulateur de débit est une pompe alimentée électriquement.

Selon un mode de réalisation, la substance présente une température d'ébullition comprise entre 30°C et 400°C à la pression atmosphérique.

Selon un mode de réalisation, la substance présente une viscosité supérieure à 1 cPa.s à 25°C, par exemple supérieure à 8 cPa.s à 25°C, et inférieure à 1 cPa.s à 60°C.

Selon un mode de réalisation, la substance est une solution comportant au moins un composé pris dans le groupe formé par les agents odorifères utilisables pour l'homme ou l'animal, les substances sémiochimiques, les agents cosmétiques, les huiles essentielles, les parfums et les agents phytosanitaires et agricoles.

Selon un mode de réalisation, les agents odorifères utilisables pour l'animal sont choisis parmi les acides gras ou la forme estérifiée desdits acides gras telle que l'oléate de méthyle, le palmitate de méthyle, l'azélate de diméthyle, et le pimelate de diméthyle.

Selon un mode de réalisation, la substance est une solution renfermant au moins une substance sémiochimique, au moins une phéromone, une allomone ou une kairomone, d'origine naturelle ou synthétique.

Selon un mode de réalisation, la substance est une solution renfermant au moins une phéromone sexuelle ou non, une allomone, une synomone ou une kairomone destinée à provoquer une réponse positive ou négative relativement à l'espèce visée, dont le résultat comportemental peut être une confusion sexuelle, une confusion d'autre nature, une attraction sexuelle, une attraction d'autre nature, une répulsion de toute nature, chez les arthropodes, dont les arachnides, ou dont les hexapodes, parmi lesquels notamment les insectes, dont les insectes nuisibles. Selon un mode de réalisation, la substance est une solution renfermant au moins une phéromone ou une phéromone sexuelle, une allomone, une synomone ou une kairomone destinée à provoquer une réponse positive ou négative relativement à l'espèce visée, dont le résultat comportemental peut être notamment un apaisement, une relaxation, une euphorisation ou une intimidation chez les classes mammalia et aves.

Selon un mode de réalisation, la substance comprend un solvant choisi parmi le myristate d'isopropyle, le dipropylène glycol, Éther de dipropylène glycol monométhylique, et un hydrocarbure isoparaffinique, par exemple une isoparaffine L ou P ou N ou V.

Selon un mode de réalisation, l'appareil comporte une pluralité de récipients de stockage contenant chacun une substance sous forme liquide ou plusieurs substances sous forme liquide et miscibles entre elles.

Selon un mode de réalisation, tout ou partie de l'ensemble des récipients de stockage est porté extérieurement par la canalisation du système d'aération.

Selon un mode de réalisation, tout ou partie de l'ensemble des récipients de stockage peut être porté extérieurement par la canalisation du système d'aération ou sa tubulure de prolongation.

Selon un mode de réalisation, chaque récipient de stockage est associé à un corps poreux de l'organe distributeur, l'ensemble des corps poreux étant mis en place à l'intérieur de la canalisation du système d'aération et étant disposés avec des décalages des corps poreux, dans une direction longitudinale de la canalisation. Selon un mode de réalisation, l'ensemble des corps poreux est mis en place à l'intérieur de la canalisation ou de la tubulure du système d'aération et pouvant être disposé avec des décalages appropriés des corps poreux pour éviter une obstruction gênante pour le passage du flux d'air.

Il est aussi décrit un procédé d'utilisation de l'appareil, dans lequel on oriente l'axe de la canalisation du système d'aération en direction et/ou en inclinaison pour atteindre une zone que l'on désire traiter.

Pour mieux faire comprendre la présente invention, on va en décrire maintenant à titre d'exemples purement illustratifs et non limitatifs, des modes de réalisation représentés sur les dessins annexés.

Sur ce dessin :
- la figure 1 représente, en perspective avec arrachement, un premier mode de réalisation de l'appareil selon l'invention ;
- la figure 2 représente une perspective analogue à la figure 1 d'un appareil selon une variante de réalisation pouvant disperser une pluralité de liquides dans le même flux d'air puisé ;
- la figure 3 représente une vue en perspective extérieure d'un appareil selon un deuxième mode de réalisation ;
- la figure 4 représente une coupe de l'appareil de la figure 3 selon le plan de coupe II-II positionné sur la figure 5 ;
- la figure 5 représente une vue en coupe selon un plan horizontal positionné selon sa trace III-III sur la figure 4 ;
- la figure 6 est un simple schéma de détail du montage de l'organe perforateur solidaire du corps poreux ;
- la figure 7 représente l'écoulement de la substance dans l'appareil de la figure 4 ;
- la figure 8 représente l'écoulement de l'air dans l'appareil de la figure 4 ;
- la figure 9 représente un corps poreux avec les organes de chauffage selon un autre mode de réalisation ;
- la figure 10 représente une vue en perspective extérieure d'un appareil selon un troisième mode de réalisation ;
- la figure 11 représente une vue écorchée de l'appareil de la figure 10 ;
- la figure 12 représente un récipient de stockage dont une zone d'étanchéité est réalisée par un joint ;
- la figure 13 représente un récipient de stockage dont une zone d'étanchéité est réalisée par une éponge ;
- la figure 14 représente un récipient de stockage, selon un mode de réalisation, totalement fermé ;
- la figure 15 représente un récipient de stockage selon un mode de réalisation à double réservoirs ;
- la figure 16 est une vue de détails agrandie qui représente l'insertion du récipient de stockage dans l'appareil avant ouverture d'un clapet du récipient de stockage par l'aiguille de l'organe distributeur ;
- la figure 17 est une vue analogue à la figure 16 qui représente l'insertion du récipient de stockage après ouverture du clapet.

Dans les modes de réalisation décrit ci-dessous, on envisage de diffuser, dans un flux d'air, une substance constituée d'une solution de phéromone répondant à la composition suivante :
- 87% en poids de (8E,10E)-dodeca-8,10-dien-1-ol, connu sous le nom de Codlémone, phéromone associée à *Cydia pomonella* (lépidoptères, tortricidés), et
- 13% en poids de dodécane-1-ol. Cette solution est connue sous le nom commercial RAK3^{®}.

La température d'entrée en ébullition à la pression atmosphérique de la solution de Codlémone est d'environ 270°C, et la viscosité à température ambiante soit 25°C, est d'environ 8 cPa.s et de 1 cPa.s à 60°C.

Il est possible de substituer, dans la solution, la Codlémone par la phéromone de formule (7E,9Z)-Dodéca-7,9-dienylaceteate, nom commercial RAK2^{®}, dont la température d'ébullition est 300°C environ. La viscosité de la solution reste proche de celle de la solution de Codlémone. La phéromone RAK2^{®} peut aussi être employée pure (100% en poids).

Alternativement, le substance peut être constituée des huiles de Colza dont la viscosité est de 7,78 cPa.s à 20°C et de 2,57 cPa.s à 50°C. La température d'ébullition est 150°C environ.

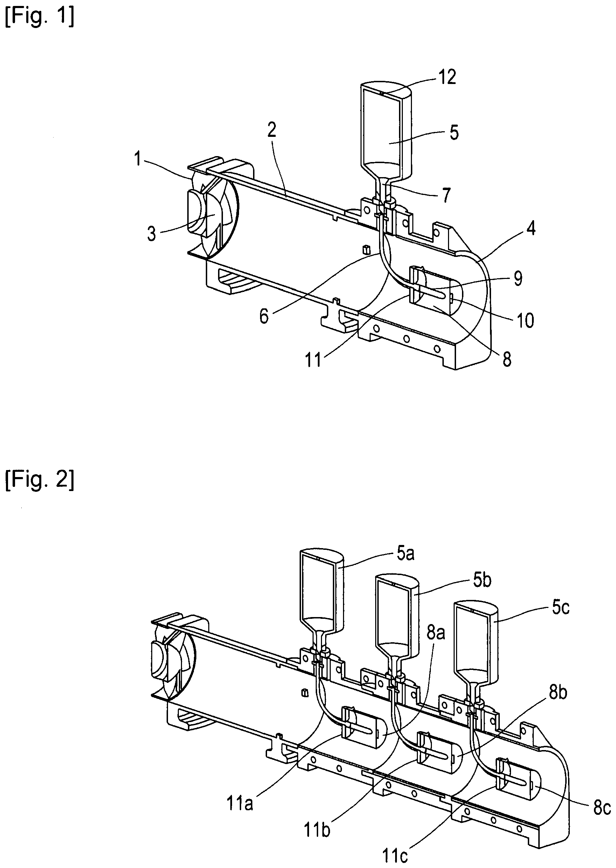

Selon un premier mode de réalisation illustré en figure 1, l'appareil est constitué d'un système de ventilation comportant un ventilateur électrique 1, dont la sortie s'effectue dans l'axe d'une canalisation cylindrique 2, le flux d'air pulsé par le ventilateur électrique 1 traversant une grille 3. Les éléments constitutifs de cette grille peuvent être profilés pour agir sur l'écoulement d'air à l'intérieur de la canalisation 2. Dans le prolongement de la canalisation 2, on a mis en place une tubulure 4 de même diamètre que la canalisation 2, à laquelle elle se raccorde. La tubulure 4 débouche à l'air libre du côté opposé à sa zone de raccordement avec la canalisation 2.

Extérieurement, la tubulure 4 porte un récipient de stockage 5, qui est destiné à recevoir la substance, dont on veut assurer la diffusion dans le flux d'air pulsé par le ventilateur électrique 1. Le récipient de stockage 5 comporte une sortie ménagée dans sa paroi, qui repose sur la tubulure 4 ; cette sortie alimente une tuyauterie 6 présentant un diamètre interne d'environ 800µm ; la tuyauterie a une longueur d'environ 3 cm ; l'entrée de la tuyauterie 6 est équipée d'une électrovanne 7, qui permet un arrêt du système, en cas d'urgence notamment. La tuyauterie 6 relie le récipient de stockage 5 à un corps poreux cylindrique en céramique 8, qui comporte un évidement cylindrique axial aveugle 9, à l'intérieur duquel l'extrémité de la tuyauterie 6 est engagée de façon étanche. Sur celle des faces d'extrémité du corps poreux 8, où ne s'effectue pas l'introduction de la tuyauterie 6, on a placé une pastille-thermomètre 10, qui est susceptible de mesurer et transmettre la température du corps poreux 8. Ce cylindre 8 porte, sur celle de ses faces, qui est opposée à celle où se trouve la pastille-thermomètre 10, un organe de chauffage 11. Le corps poreux 8 est en alumine et présente des pores de 100 nm de diamètre et une porosité uniforme de 40%.

Sur la surface du récipient de stockage 5, on met en place un marquage électronique 12, qui permet d'identifier la solution de Codlémone placée dans le récipient 5. Ce marquage électronique prend la forme d'une étiquette comprenant une puce de radio-identification, aussi appelé puce RFID (de l'acronyme anglais « radio frequency identification). En partie haute du récipient 5, on a ménagé une ouverture étanche au liquide permettant le maintien de l'intérieur du récipient à la pression atmosphérique. Le corps poreux 8 est choisi en fonction de la substance à diffuser, ici la Codlémone. Il est possible que le corps poreux 8 et la tuyauterie 6 puissent être constitués d'une pièce unique et/ou d'être venu de matière.

Les informations relatives aux caractéristiques propres à la substance, aux caractéristiques choisies pour le corps poreux 8 et/ou à la température du corps poreux 8, sont des informations, qui sont envoyées sur un contrôleur électronique (non représenté), qui assure, de façon automatique, les quelques réglages utiles pour ajuster à la valeur souhaitée le rapport des débits d'air, c'est-à-dire le rapport entre le débit d'air sans le ventilateur électrique et le débit d'air généré par le ventilateur, et la température du corps poreux 8 quantifiant le débit évaporé de la solution de phéromone dans le flux gazeux produit par l'appareil selon une des variantes du procédé de pilotage décrit.

La substance est tractée dans la tuyauterie 6 par une force de pompage capillaire générée par le fait que la substance se déplace dans des micro-canalisations vis-à-vis des parois desquelles la substance est mouillante en raison de sa tension de surface. Bien entendu, les matériaux utilisés sont suffisamment neutres pour ne pas dégrader le mélange à long terme et pour que les tensions de surface ne soient pas modifiées. La force capillaire est provoquée par la nature de la surface, qui est constituée de canaux ou de pores suffisamment étroits pour générer une traction capillaire ; le liquide est mouillant en regard des matériaux de la tuyauterie 6 et du corps poreux 8. Le liquide affleure ainsi à l'extrémité des pores du corps poreux, dont l'ensemble constitue la surface d'évaporation ainsi située à la périphérie du corps poreux 8.

Il faut que la force de traction et de rétention capillaire permette un affleurement du liquide à l'extrémité des pores de la surface d'évaporation ; néanmoins, cet affleurement doit s'effectuer sans toutefois permettre un étalement incontrôlé sur la surface d'évaporation via les forces dues aux champs de gravité (attraction terrestre et pression hydrostatique de la colonne de liquide potentiellement présente) ou aux forces d'attraction statique générées par les interactions entre la solution et le reste de la surface de la mèche. Cette traction capillaire n'existe que par renouvellement de ce bloc volume final (la section/cylindre de liquide à l'extrémité du pore). Le renouvellement de ce volume est effectué par l'évaporation et est régi par l'équilibre des concentrations des molécules liquides et gazeuses à l'interface liquide et gaz selon une grandeur propre à chaque solution et dépendante principalement de la température (à pression atmosphérique), à savoir, la pression de vapeur saturante. L'augmentation de la température de la solution à évaporer entraine une augmentation de la pression de vapeur saturante, donc le déplacement de l'équilibre des concentrations de molécules liquides et gazeuses à l'interface vers les molécules gazeuses : il y a évaporation jusqu'au nouvel équilibre. Si la phase gazeuse est mobile, l'équilibre n'est jamais atteint et l'évaporation se poursuit jusqu'à épuisement de la phase liquide. Plus la phase gazeuse est mobile (et tend plus vite à évacuer les molécules en phase gaz) plus l'évaporation est rapide.

On a constaté que, dans un système du type de celui précédemment décrit, la cinétique d'évaporation est multipliée par un facteur compris entre 1 et 10 quand on passe de 0 à 24 m/s de ventilation ; en outre, si on passe le liquide de 20°C à 70°C, on augmente la cinétique d'évaporation en la multipliant par un facteur compris entre 20 et 100.

Le réglage des paramètres du système décrit, peut s'effectuer en agissant sur le ventilateur 1 (action sur le débit d'air) et/ou en agissant sur l'organe de chauffage, ici un réchauffeur électrique 11, aussi appelé résistance, placé sur la surface d'évaporation. La mesure, que l'on peut effectuer au moyen du thermomètre 10, permet de régler l'intensité ou le temps d'activation du réchauffeur électrique pour obtenir la température souhaitée de la surface d'évaporation souhaitée. On peut aussi prévoir à l'extrémité libre de la tubulure 4, des perturbateurs du flux d'air soufflé ou des convecteurs pour ajuster la surface sur laquelle la substance est dispersée.

La figure 2 représente une variante de réalisation de l'appareil dans laquelle ledit appareil est équipé de trois récipients de stockage distincts 5a, 5b, 5c, respectivement associés à des organes distributeurs constitués de corps poreux 8a, 8b, 8c, tout à fait similaires au corps poreux 8 précédemment décrit pour la variante de la figure 1. A chaque corps poreux est associé un réchauffeur électrique 11a, 11b, 11c, placé sur la surface extérieure du corps poreux. Les corps poreux 8a, 8b, 8c, sont décalés les uns par rapport aux autres dans la voie de soufflage d'air, qui est définie par la tubulure 4, de sorte que le fait d'avoir augmenté le nombre de corps poreux, évite de constituer une obstruction gênante pour le passage de l'air. Sur la figure 2, les corps poreux 8a, 8b, 8c sont placés en série, toutefois dans une variante de réalisation non représentée, les corps poreux peuvent être disposés en parallèle.

L'utilisateur de l'appareil, qu'il s'agisse d'un appareil du type de la figure 1 ou de la figure 2, va donc agir sur le fonctionnement par action sur la température du ou des corps poreux 8, 8a, 8b, 8c, par action sur les résistances associées aux corps poreux et par action sur la vitesse de ventilation (alimentation électrique du ventilateur 1). Toutes ces fonctions peuvent être facilement regroupées sur un contrôleur (non représenté) et l'appareil selon l'invention a donc un fonctionnement, qui peut être rendu entièrement automatique, le marquage électronique 12 permettant de différencier les liquides à diffuser. Le contrôleur peut posséder une antenne de connexion, qui permet le transfert des informations du contrôleur vers l'utilisateur ou inversement. Alternativement, le fonctionnement peut être piloté à distance par l'utilisateur via un téléphone intelligent par exemple.

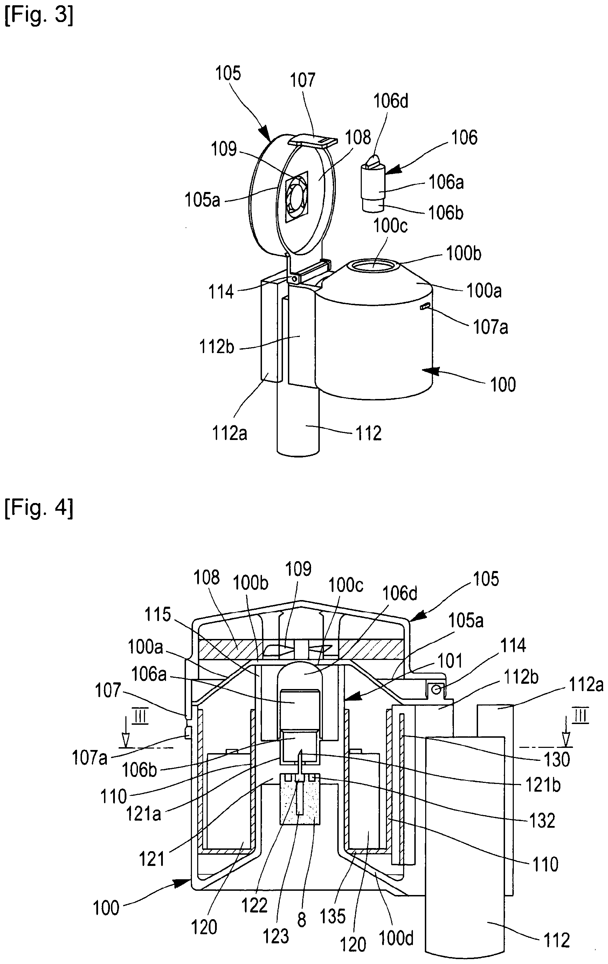

Selon un deuxième mode de réalisation illustré sur la figure 3, l'appareil comporte un carter cylindrique d'axe vertical désigné par 100 dans son ensemble ; ledit carter est supporté, à environ 1,50m du sol, par un pied 112, au sommet duquel il s'accroche mécaniquement par deux mâchoires serrables 112a, 112b ; la mâchoire 112b est solidaire du carter 100. La partie supérieure du carter 100 a la forme d'un tronc de cône 100a, dont la bordure supérieure 100b délimite une ouverture circulaire 100c du côté opposé au sol. La paroi tronconique 100a est susceptible d'être recouverte par un couvercle désigné par 105 dans son ensemble ; le couvercle 105 est articulé au moyen d'un axe 114 sur la mâchoire 112b ; l'axe 114 est perpendiculaire à l'axe du pied 112.

Le couvercle 105, lorsqu'il est ouvert comme représenté sur la figure 3, dégage totalement l'orifice 100c et permet d'introduire, dans le carter 100, un récipient de stockage cylindrique désigné par 106 dans son ensemble. Le récipient 106 renferme la substance liquide, dont on désire assurer la diffusion à l'état vapeur dans l'air ambiant. Le récipient 106 comporte deux parties : la partie supérieure 106a est réalisée en matière plastique résistante, alors que la partie inférieure 106b comporte une paroi facilement perforable. Le récipient 106 est muni, à sa partie supérieure, d'une languette de préemption 106d.

En référence à la figure 4, lorsque le couvercle 105 est en position fermée, la position du couvercle par rapport au carter 100 est maintenue au moyen d'un élément de fermeture 107, solidaire du couvercle 105. L'élément de fermeture 107 coopère avec un encliquetage approprié 107a du carter 100. Un élément du couvercle 105 vient en appui sur la partie 106a du récipient 106 pour appliquer le fond de la partie 106b contre le fond d'un logement 121, qui sera décrit plus loin. Lorsque le couvercle 105 est en position fermée, sa bordure inférieure 105a se trouve au droit du tronc de cône 100a, qui forme la partie haute du carter 100 ; mais elle laisse subsister un espace libre entre le bas du couvercle 105 et le tronc de cône 100a. Dans le fond du couvercle 105, on a mis en place un filtre 108 ayant la forme d'une collerette plane circulaire, de même axe que le couvercle 105 ; lorsque le couvercle 105 est fermé, l'axe de la collerette-filtre 108 devient celui du carter 100. Dans l'évidement central ménagé dans la collerette-filtre 108, on a mis en place un composant ventilateur 109, qui est alimenté électriquement par un conducteur (non représenté) porté par la paroi du couvercle 105. L'air est aspiré par le ventilateur 109 à travers l'espace ménagé entre le couvercle 105 et le tronc de cône 100a ; il traverse alors la collerette-filtre 108 et vient au droit de l'ouverture circulaire 100c. Le carter 100 comporte intérieurement une structure 101, qui relie le tronc de cône 100a de sa partie supérieure à un évasement tronconique100d, qui constitue la base inférieure du carter 100. Entre la partie de plus petite section de l'évasement 100d et la partie de plus petite section de la bordure 100b de l'orifice 100c, on a ménagé une paroi cylindrique 115, à l'intérieur de laquelle, sensiblement à mi-hauteur, on a disposé un croisillon 121 prévu pour supporter, dans sa partie centrale, le récipient 106. La partie centrale du croisillon 121 comporte un logement 121a ouvert en direction du couvercle 105 ; dans ce logement, se positionne la partie 106b du récipient de stockage 106. Le fond du logement 121a comporte un organe perforateur en relief 121b, constitué par une aiguille 133, dont l'extrémité est découpée en biseau : cette aiguille est susceptible de perforer le fond de la partie 106b du récipient de stockage 106 lorsque celui-ci est positionné par un opérateur dans son emplacement 121a prévu. L'aiguille 121b définit un passage capillaire 134 en direction d'un corps poreux 8 de forme cylindrique, constitué d'alumine frittée. Le corps poreux 8 présente des pores de 100 nm de diamètre et une porosité uniforme de 40%. L'aiguille 121b est enfoncée dans un avant-trou 122, de façon étanche et maintenu par collage, et elle alimente une canalisation aveugle 123 pratiquée selon l'axe longitudinal du corps poreux 8.

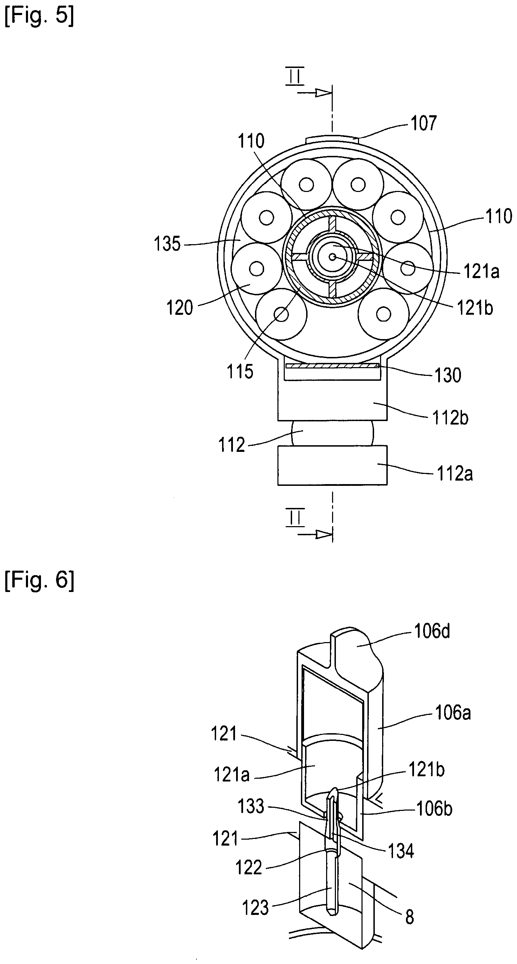

Autour de la partie centrale de la structure, qui vient d'être décrite et qui a été désignée de façon générale par la référence 101, se trouve une autre paroi cylindrique 110 coaxiale à la paroi cylindrique qui délimite la zone du réservoir de stockage 106 et s'étend autour du corps poreux 8. Cette paroi cylindrique 110 est solidaire d'un fond, qui constitue une collerette 135 reliant entre elles les deux parois cylindriques 110 et 115 ; sur cette collerette 135, on a disposé des piles électriques 120 régulièrement réparties autour de l'axe du carter 100 ; l'ensemble (110,115,135) constitue un barillet, comme il est bien visible sur la figure 5. Les piles 120 fournissent l'énergie nécessaire au fonctionnement de l'appareil selon l'invention.

Ces piles sont reliées à une carte de commande 130, qui est logée dans la partie de la mâchoire 112b placée tangentiellement par rapport au barillet de piles. La carte 130 est reliée électriquement, d'une part, au moteur du ventilateur 109 et, d'autre part, à des organes chauffants 132 insérés dans le corps poreux 8, notamment sur sa face qui est insérée à l'intérieur des bras radiaux du croisillon 121.

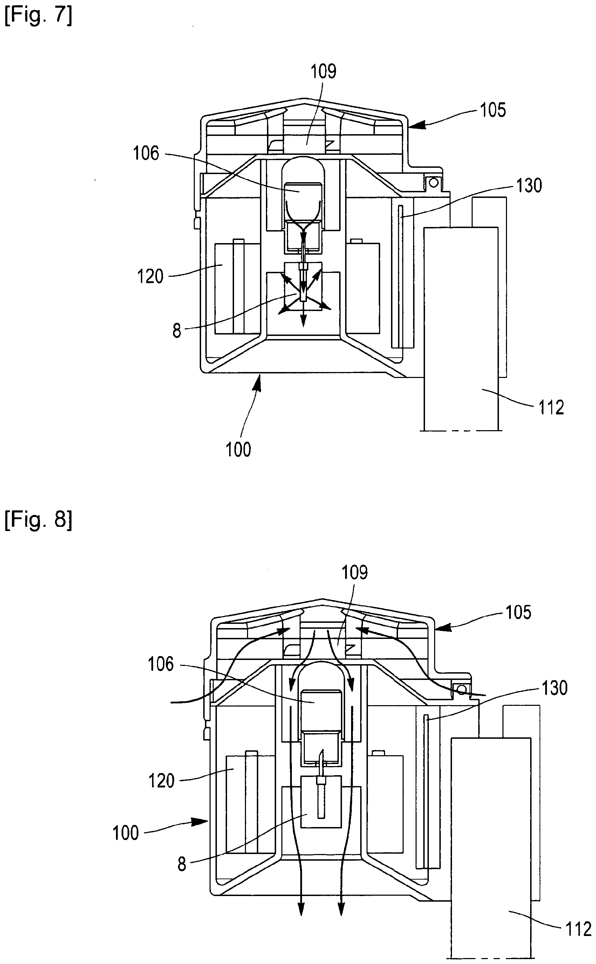

Dans l'appareil, qui vient d'être décrit, la solution de Codlémone amenée par le récipient de stockage 106, se répartit, dès que le couvercle 105 a provoqué la perforation du récipient 106b par l'élément perforateur 121b, à travers le corps poreux 8, dont la zone d'évaporation est la surface libre comme indiqué par des flèches sur la figure 7.

En référence à la figure 8, l'air, qui assure l'évaporation, pénètre sous le couvercle 105, dans lequel il est aspiré par le ventilateur 109 ; cet air s'écoule autour du récipient de stockage 106, traverse le croisillon 121 et s'évacue à l'extérieur en passant par l'évasement tronconique 100d, après s'être chargé de la vapeur de la solution de Codlémone au niveau de la zone d'évaporation que constitue la surface libre du corps poreux 8. L'écoulement de l'air est matérialisé par des flèches.

Le débit d'air et la température du corps chauffant sont régulés par la carte de commande 130.

De préférence, la substance et le corps poreux 8 ont des propriétés physiques qui permettent une régulation du débit par contrôle de température dans le corps poreux 8.

En particulier, dans un mode de réalisation préféré :
- aucun écoulement substantiel ne se produit à température ambiante, c'est-à-dire par exemple dans une plage de température comprise entre 0°C et 30°C,
- l'écoulement et l'évaporation se produisent au-dessus d'une température de consigne T qui peut être atteinte par les organes chauffants 132

La carte de commande 130 pilote les organes chauffants 132 en fonction d'un programme de commande stockée dans sa mémoire. Ce programme définit par exemple les heures de début et de fin de distribution, les températures de consigne, les débits d'air (si ventilation forcée), etc.

Dans un mode de réalisation non représenté, l'électrovanne des premier et deuxième modes de réalisation peut être remplacée par une vanne manuelle. Elle peut aussi être supprimée dans chacun des modes de réalisation.

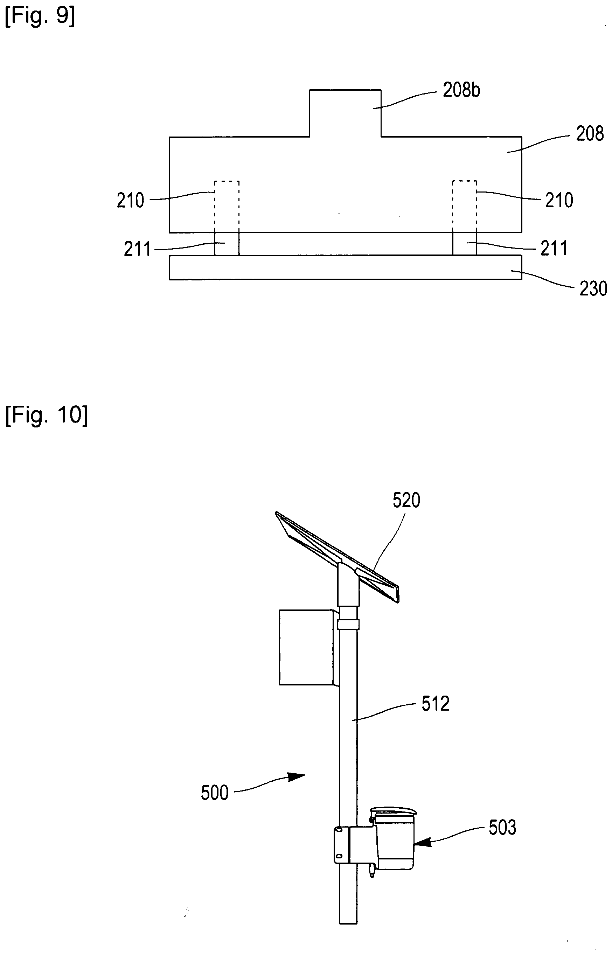

Une variante de réalisation du corps poreux est illustrée sur la figure 9. Le corps poreux 208 présente un forme cylindrique surmonté d'un ergot 208b. Cet ergot permettra de conduire la substance vers le reste du corps poreux lorsque la cartouche sera montée dans l'appareil. Sur la face du corps poreux opposée celle portant l'ergot, deux évidements 210 sont pratiqués afin d'accueillir chacun un organe de chauffage 211. Les organes de chauffage 211 sont des résistances électriques alimentés par un circuit électrique 230.

Dans cette variante de réalisation, le corps poreux peut aussi bien avoir une porosité uniforme que non-uniforme. Dans ce dernier cas, la porosité ouverte est de 25% au cœur et de 45 % en surface. Ce sera alors un corps poreux dont la porosité ouverte, c'est-à-dire le volume des pores par unité de volume du corps poreux, augmente du cœur vers la surface d'évaporation. Ainsi on privilégie un étalement le plus grand possible sur l'intégralité de la surface du corps poreux à la sortie des pores, et on préserve l'intégrité mécanique du cœur poreux avec un cœur plus dense.

Un troisième mode de réalisation de l'appareil est illustré sur la figure 10. L'appareil 500 comporte un carter d'axe vertical 503 ; ledit carter est supporté, à environ 1,50m du sol, par un pied 512, au sommet duquel sont fixés des panneaux solaires 520 afin d'alimenter en énergie l'appareil 500. Le carter 503 est mécaniquement attaché au pied par deux mâchoires serrables 512a, 512b ; la mâchoire 512b est solidaire du carter 503. De préférence, une articulation (non représentée) est agencée entre la machoire 512b et le carter 503 pour permettre un réglage d'orientation du carter 503.

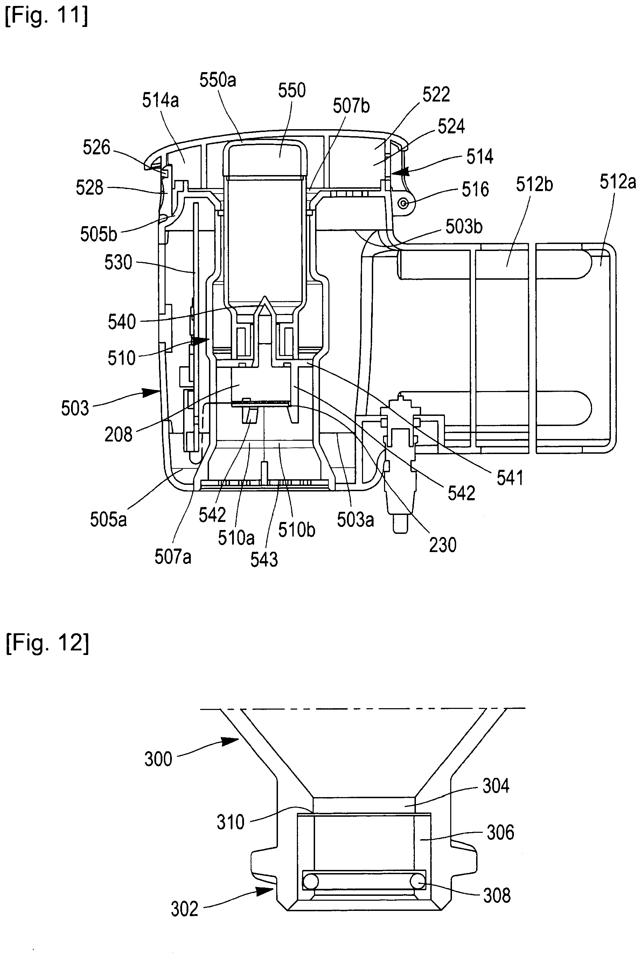

En référence à la figure 11, le carter 503 présente la forme d'un cylindre de directrice carrée. La bordure supérieure 503b du carter délimite une ouverture supérieure carrée aux coins arrondis du côté opposé au sol, et la bordure inférieure 503a du carter délimite une ouverture inférieure carrée aux coins arrondis du côté face au sol. L'ouverture supérieure est recouverte de manière étanche d'une pièce supérieure 505b et l'ouverture inférieure est recouverte de manière étanche d'une pièce inférieure 505a. Les pièces supérieure et inférieure comportent une ouverture centrale chacune 507a,507b, les deux ouvertures centrales ayant le même axe central.

La pièce supérieure 505b est susceptible d'être recouverte par un couvercle 514 ; le couvercle 514 est articulé au moyen d'un axe 516 perpendiculaire à l'axe du pied 512.

Le couvercle 505, lorsqu'il est ouvert dégage totalement l'ouverture centrale 507b et permet d'introduire, dans le carter 503, un récipient de stockage cylindrique désigné par 550 dans son ensemble. Le récipient 507 renferme la solution de phéromone, dont on désire assurer la diffusion à l'état vapeur dans l'air ambiant. Lorsque le couvercle 514 est en position fermée comme illustré sur la figure 11, la position du couvercle par rapport au carter 503 est maintenue au moyen d'un élément de fermeture 526, solidaire du couvercle 514. L'élément de fermeture 526 coopère avec un encliquetage approprié 528 de la pièce supérieure 505b. Un élément du couvercle 514 vient en appui sur la partie 550a du récipient 550 pour que l'aiguille 540 perce le bouchon du récipient 550 et maintenir le récipient en position dans le carter. Lorsque le couvercle 514 est en position fermée, sa bordure inférieure 514a se trouve au droit des parois latérales de la pièce supérieure 507b, qui forme la partie haute du carter 503. La bordure inférieure 514a présente une ouverture 522 de manière à laisser circuler de l'air dans le carter 503. Pour éviter que des poussières ne rentrent par l'ouverture 522, un filtre 524 est placé derrière l'ouverture.

Le carter 503 comprend en outre un cylindre creux 510 formé de deux demi-cylindres creux 510a, 510b identiques. Ces deux demi-cylindres prennent en sandwich lorsqu'ils sont assemblés le corp poreux 208 surmonté d'une aiguille 540 et reposant sur l'organe de chauffage, dont on a représenté le circuit électrique 230. L'aiguille est fixée au corps poreux grâce des clips 542 s'étendant longitudinalement depuis une collerette 541 à la base de l'aiguille 540. Les deux demi-cylindres prennent aussi en sandwich lorsqu'ils sont assemblés, un filtre 543 au niveau de leur base, et deux ventilateurs (non représentés) au niveau de la jonction des paroi latérales des demi-cylindres. Le maintien de l'ensemble formé par l'aiguille et le corps poreux s'effectue par une rainure à l'intérieur des parois du cylindre, la rainure accueillant la collerette 541. Le filtre est fixé au cylindre de manière identique. Enfin le cylindre 510 est maintenu entre les pièces supérieure 507b et inférieure 507a au droit des ouvertures de ces pièces 507b, 507a, les pièces supérieures et inférieures prenant en sandwich le cylindre 510.

Les panneaux solaires sont reliés reliées à une carte de commande 530, qui est logée dans un logement entre les parois du carter 503, le cylindre creux 510 et les pièces supérieure et inférieure. La carte 530 est reliée électriquement, d'une part, aux ventilateurs et, d'autre part, à l'organe de chauffage, dont on a représenté le circuit électrique 230.

En référence à la figure 12, le récipient de stockage 300 présente une ouverture 304 dans sa partie inférieure 302. L'ouverture est équipée d'un bouchon afin d'empêcher la substance de couler lorsque le récipient de stockage n'est pas utilisé. Ce bouchon est constitué d'une bague 306 supportant un joint torique 308, et d'une membrane 310 collée sur la bague. La membrane comprend une feuille d'aluminium étanche et perforable ou déplaçable à la manière d'un clapet.

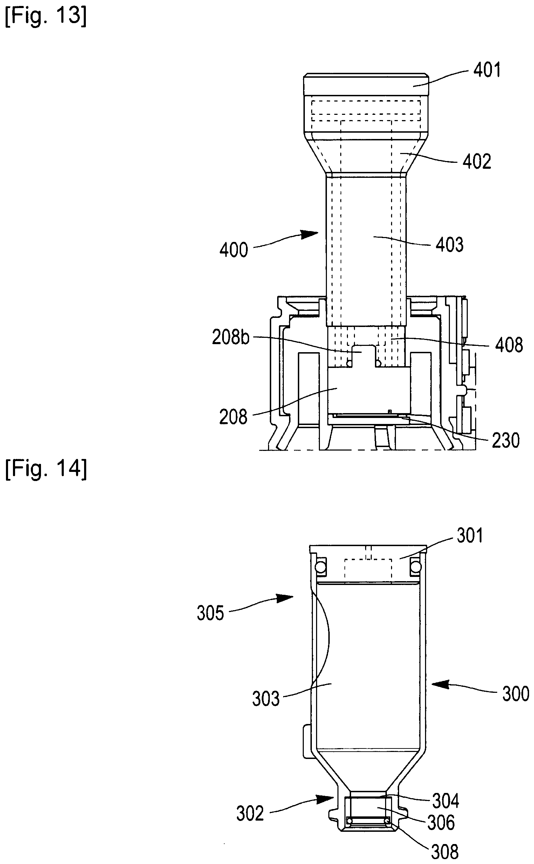

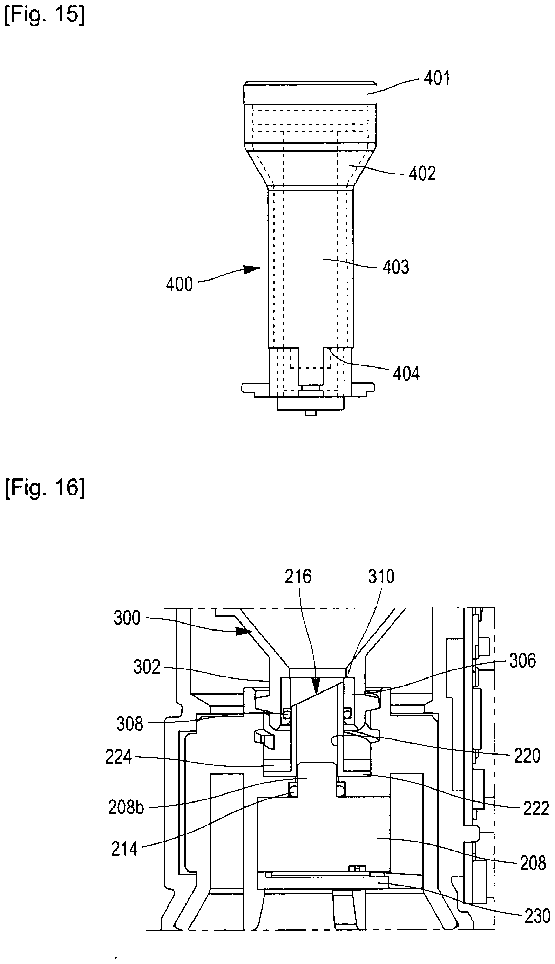

Le récipient de stockage peut être prévu pour être amovible notamment parce que cela facilite le changement de récipient de stockage à moindre coûts. Selon un mode de réalisation non représenté, le bouchon comprend alors en outre un clapet configuré pour se refermer lorsque le récipient de stockage est retiré de l'appareil. Dans ce cas-là, il est impossible de retirer le récipient de stockage tant que tout le corps poreux n'est pas imbibé de la substance contenue dans le corps poreux. Alternativement à l'utilisation d'une aiguille et d'un clapet, le récipient de stockage peut contenir une éponge comme illustré sur les figures 13 et 15. L'ergot 208b du corps poreux entre en contact avec un autre corps poreux formant organe de rétention, ici une éponge 408, contenu dans le récipient de stockage et en constituant une des extrémités libres. L'éponge 408 est alors compressée par le corps poreux 208 pour assurer un bon contact. Le transfert d'un corps poreux 208b à l'organe de rétention par contact et par traction capillaire peut s'effectuer. Le récipient de stockage est dès lors amovible et le liquide ne s'écoulera pas du récipient lorsque le contact sera rompu avec le corps poreux 208b, de la même manière que lors du fonctionnement à froid (température ambiante), le liquide ne s'écoule pas du corps poreux 208 . Cette éponge 408 est généralement en feutre de laine ou en mélamine. En conclusion, l'éponge est de préférence souple et légèrement compressible par le corps poreux 208 pour assurer le contact.

De manière générale, le maintien du récipient de stockage à l'appareil est assuré par pression, par exemple grâce à des clips, ou par vissage de la tête du récipient de stockage. Dans tous les cas, le contact entre le récipient de stockage et le corps poreux est étanche en raison de la présence d'un joint.

Pour que l'adhérence de la substance au corps poreux 208 soit suffisante, un des paramètres à contrôler est la pression à l'intérieur du récipient de stockage. En effet, si le récipient de stockage est ouvert à l'air libre, l'adhérence de la substance ne sera jamais suffisante pour compenser la force de gravité s'exerçant sur le liquide. Il faut donc gérer cette force de gravité. Deux types de récipients de stockage peuvent être utilisés. Le premier type de récipients de stockage est un réservoir totalement fermé sauf en une de ses extrémités qui est en contact avec le corps poreux. Ce type de récipients de stockage est illustré sur la figure 14. Le récipient de stockage 300 comprend un unique réservoir 303 surmonté d'une fermeture étanche 301. La partie inférieure 302 du récipient de stockage comprend un bouchon tel que décrit en figure 12. A chaque goutte écoulée vers le corps poreux, la dépression augmente dans partie haute 305 du récipient de stockage c'est-à-dire la partie où il n'y a pas ou plus de liquide. Pour que l'écoulement s'effectue totalement, il faut laisser dès la mise en place du récipient de stockage 300 dans l'appareil un volume sans liquide suffisamment grand dans le réservoir, soit environ un volume de 40% par rapport au volume total du réservoir. Ainsi, la dépression va augmenter au fur et à mesure et empêcher l'écoulement libre, mais ne sera jamais suffisante pour bloquer la totalité de l'écoulement vers la surface du corps poreux.

En référence à la figure 15, le récipient de stockage 400 comprend un réservoir extérieur 402 totalement clos si ce n'est à son extrémité en contact avec le réservoir intérieur 403. Le réservoir intérieur 403 est surmonté d'un évent 401 en son extrémité supérieure, l'évent permettant l'équilibre des pressions entre l'air extérieur et l'intérieur du réservoir intérieur. Le réservoir intérieur 403 est en contact avec le corps poreux en son extrémité inférieure. Ainsi, à chaque goutte écoulée vers le corps poreux, le réservoir intérieur 403 s'équilibre par son évent 401, et entraine une baisse du niveau. Par vase communicant via la jonction 404 entre les deux réservoirs, le réservoir extérieur 402 remplit le réservoir intérieur 403, mais alors la dépression du réservoir extérieur 402 augmente dans la partie du réservoir où il n'y a pas ou plus de liquide. Ainsi on équilibre le réservoir intérieur 403 avec la dépression du réservoir extérieur 402. Le réservoir intérieur 403 peut toutefois toujours sortir de cet équilibre grâce à son évent 401 et la traction réalisée par le corps poreux de l'organe distributeur. Pour que l'écoulement puisse s'effectuer normalement, lors de la mise en place du récipient de stockage 400 dans l'appareil, le réservoir extérieur 402 est complètement rempli de la substance.

L'organe de rétention décrit plus haut peut aussi être employé dans le récipient de stockage 400. Dans le récipient de stockage 400, l'organe de rétention, par exemple en éponge ou mousse alvéolaire, peut occuper tout ou partie du réservoir intérieur 403.

En référence à la figure 16, le dispositif pour disperser la substance contenue dans le récipient de stockage 300 comprend le corps poreux 208 précédemment décrit dont la base coopère avec l'organe de chauffage, dont on a représenté le circuit électrique 230. L'ergot 208b du corps poreux est surmonté d'une aiguille creuse 220, l'ergot 208b s'emboitant dans la base 222 de l'aiguille. La base 222 s'étend radialement jusqu'à couvrir la surface supérieure du corps poreux. Afin d'assurer une liaison étanche entre l'ergot et l'aiguille, un joint torique 214, entourant complètement l'ergot, est placé entre l'ergot et l'aiguille. La partie supérieure 216 de l'aiguille prend la forme d'un biseau afin de percer plus facilement le bouchon du récipient de stockage tel que décrit sur la figure 12 et sur la figure 14. Le récipient de stockage est 300 est introduit dans le dispositif par sa partie inférieure 302. Le maintien du récipient de stockage dans le dispositif s'effectue par vissage. Lorsque le vissage de la partie inférieure 302 du récipient commence, l'aiguille pénètre dans la bague 306 puis entre en contact latéralement avec le joint torique 308 supporté par la bague de manière à ce que la liaison entre l'aiguille et le bouchon soit étanche. Puis au fur et à mesure que le vissage continue, l'aiguille se rapproche de le membrane 310 collée sur la bague.

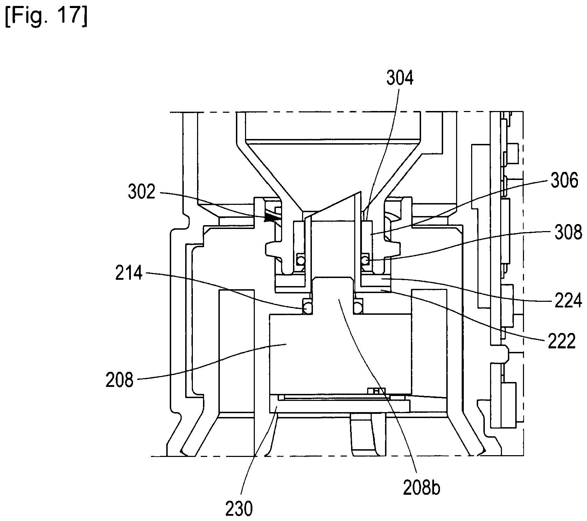

A la fin du vissage, le biseau de l'aiguille déplace réversiblement la membrane 310 à la manière d'un clapet, comme illustré sur la figure 17. La partie inférieure 302 du récipient vient en contact avec un joint d'étanchéité 224 placé sur l'extension radiale de la base 222 de l'aiguille. La substance peut alors s'écouler dans l'intérieur de l'aiguille. L'aiguille guide la substance jusqu'à l'ergot. La substance pourra alors emprunter les micro-canalisations du corps poreux 208 pour atteindre la surface d'évaporation.

Dans le cas où il est nécessaire de changer le récipient de stockage, par exemple parce qu'il est vide où qu'il faut changer de substance, le récipient est dévissé. Lorsque l'aiguille ne traverse plus la membrane, cette dernière se referme empêchant ainsi la substance de couler.

Dans une variante du récipient de stockage 300, l'organe de rétention alvéolaire décrit plus haut est employé à la place de la membrane 310. Dans ce cas, l'organe distributeur ne comporte pas d'aiguille mais un corps poreux qui vient en contact directement avec l'organe de rétention alvéolaire pour exercer la traction capillaire comme décrit plus haut.

### Exemple quantitatif

Dans une application agricole, une solution de codlémone est diffusée dans un milieu dont la température ambiante varie typiquement entre 0°C et 50°C, et préférentiellement entre 10°C et 45°C.

L'appareil est configuré pour que :
- avec activation du chauffage et/ou avec activation du chauffage à la température de consigne choisie, par exemple entre 50°C et 65°C, le débit de solution évaporée soit égal à un débit nominal prédéfini D, par exemple entre 1 mg/h et 100 mg/h, de préférence entre 5 mg/h et 20 mg/h
- sans activation du chauffage, c'est-à-dire à la température ambiante, le débit évaporé soit inférieur à D/10, de préférence inférieur à D/50.

Des paramètres pertinents pour régler le débit nominal D sont non seulement la température de fonctionnement et la viscosité de la solution, mais aussi des paramètres constructifs, comme le dimensionnement de l'organe distributeur, notamment l'aire de sa surface d'évaporation.

Certains des éléments décrits, notamment le dispositif de commande, les cartes de commande ou les contrôleurs électroniques, peuvent être réalisés sous différentes formes, de manière unitaire ou distribuée, au moyen de composants matériels et/ou logiciels. Des composants matériels utilisables sont les circuits intégrés spécifiques ASIC, les réseaux logiques programmables FPGA ou les microprocesseurs. Une horloge locale et/ou une horloge réseau peut être intégrée dans ces éléments pour fournir des références temporelles.

Bien que l'invention ait été décrite en liaison avec plusieurs modes de réalisation particuliers, il est bien évident qu'elle n'y est nullement limitée et d'autres équivalents techniques des moyens décrits ainsi que leurs combinaisons peuvent être imaginé(e)s dans d'autre modes de réalisation non décrits dans cette description, si celles-ci entrent dans le cadre de l'invention, lequel est défini par les revendications.

L'usage du verbe « comporter », « comprendre » ou « inclure » et de ses formes conjuguées n'exclut pas la présence d'autres éléments ou d'autres étapes que ceux énoncés dans une revendication.

Dans les revendications, tout signe de référence entre parenthèses ne saurait être interprété comme une limitation de la revendication.

## Revendications

1. Appareil pour disperser dans l'air, à l'état de vapeur, une substance à l'état liquide à température ambiante et contenue dans un récipient de stockage, l'appareil comportant :
- un système d'aération comportant une canalisation (2, 4, 510) débouchant à l'air libre et configuré pour permettre le passage d'un débit d'air dans la canalisation ;
- au moins un organe distributeur (8, 208) destiné à être alimenté en une substance liquide par le récipient de stockage, l'organe distributeur comportant des micro-canalisations formant une sortie agencée dans la canalisation afin d'y constituer une zone d'évaporation de la substance,
- au moins un récipient de stockage (5, 106, 300, 400, 550) contenant la substance et relié à l'organe distributeur (8,208), le récipient présentant un orifice de vidange relié à l'organe distributeur, et
- un organe de chauffage (11, 211, 132) agencé sur ou dans l'organe distributeur de manière à contrôler un écoulement de la substance à travers l'organe distributeur,
l'orifice de vidange est orienté vers le bas lorsque l'appareil est dans une position d'utilisation, l'organe distributeur étant situé en dessous de l'orifice de vidange dans la position d'utilisation, et en ce que ladite substance présente une viscosité variable en fonction de la température, l'appareil étant **caractérisé en ce que** ladite viscosité est telle que, dans la position d'utilisation, la substance ne peut pas s'écouler à travers les micro-canalisations de l'organe distributeur à une température ambiante inférieure à une première température, l'organe de chauffage étant configuré pour chauffer l'organe distributeur jusqu'à une deuxième température supérieure à la première température de sorte qu'un écoulement de la substance à travers les micro-canalisations de l'organe distributeur se produit par capillarité dans la position d'utilisation.

2. Appareil selon la revendication 1, dans lequel l'organe distributeur comporte un corps poreux (8, 208) comprenant des pores, lesdits pores constituant au moins une partie des micro-canalisations de l'organe distributeur.

3. Appareil selon la revendication 2, dans lequel les pores présentent un diamètre compris entre 0.01 et 10 µm.

4. Appareil selon l'une des revendications 2 à 3, dans lequel le corps poreux (8, 208) présente une forme de cylindre.

5. Appareil selon la revendication 4, dans lequel l'alimentation en substance est reçue dans un évidement aveugle (9) prévu parallèlement à l'axe du corps poreux (8).

6. Appareil selon la revendication 4, dans lequel le corps poreux comprend un ergot (208b) agencé sur une partie supérieure dudit corps poreux et s'étendant selon un axe longitudinal du corps poreux et configuré pour recevoir la substance.

7. Appareil selon l'une des revendications 2 à 6, dans lequel l'organe distributeur (8) comporte une membrane périphérique agencée autour du corps poreux et percée de trous constituant des micro-canalisations.

8. Appareil selon l'une des revendications 2 à 7, dans lequel le corps poreux (8, 208) présente une porosité dans une partie intérieure du corps poreux plus faible qu'une porosité dans une partie extérieure du corps poreux entourant la partie intérieure.

9. Appareil selon l'une des revendications 2 à 8, dans lequel le corps poreux comporte une mèche en bois, en textile, en céramique ou en polymère.

10. Appareil selon l'un des revendications 2 à 9, dans lequel l'organe de chauffage (11, 132, 211) est placé directement sur une surface du corps poreux.

11. Appareil selon l'une des revendications 2 à 9, dans lequel le corps poreux présente au moins un évidement (210) logeant au moins une partie de l'organe de chauffage.

12. Appareil selon l'une des revendications 1 à 11, dans lequel l'organe distributeur comprend une aiguille creuse (220) configurée pour percer un opercule et/ou déplacer une membrane (310) faisant clapet du récipient de stockage et amener la substance contenue dans le récipient de stockage jusqu'à la surface d'évaporation.

13. Appareil selon la revendication 12 prise en combinaison avec l'une des revendications 2 à 11, dans lequel l'aiguille (220) est disposée à une des extrémités du corps poreux.

14. Appareil selon l'une des revendications 1 à 13, dans lequel les micro-canalisations présentent une section comprise entre 10⁻⁴ µm² et 10⁶ µm², de préférence entre 0,1 µm² et 10³ µm².

15. Appareil selon l'une des revendications 1 à 14, comportant en outre un organe de fixation orientable en direction et/ou en inclinaison par rapport à la canalisation du système d'aération, afin d'orienter la canalisation par rapport au sol lorsque l'organe de fixation est fixé à un support.

16. Appareil selon l'une des revendications 1 à 15, dans lequel le système d'aération comporte au moins un ventilateur (1) mis en place dans une partie de la canalisation (2,4, 510).

17. Appareil selon l'une des revendications 1 à 16, dans lequel le système d'aération comporte des ouvertures pratiquées dans une paroi d'extrémité de la canalisation (2,4) et des obturateurs réglables équipant lesdites ouvertures pour permettre de régler une section de passage des ouvertures.

18. Appareil selon la revendication 16 ou 17, dans lequel ledit appareil comprend un organe régulateur (130, 530) d'un débit d'air dans la canalisation, configuré pour contrôler le ventilateur et/ou les obturateurs afin de réguler un débit d'air dans la canalisation.

19. Appareil selon l'une des revendications 1 à 18, comprenant en outre un dispositif de commande (130, 530) configuré pour commander l'organe de chauffage (11, 132, 211) en fonction d'une température de consigne dans l'organe distributeur

20. Appareil selon la revendication 19, dans lequel l'organe de chauffage comprend au moins une carte électronique (130, 230) et au moins une résistance électrique (211) alimentée électriquement par la carte électronique.

21. Appareil selon la revendication 20, dans lequel le dispositif de commande est agencé sur la carte électronique (130).

22. Appareil selon l'une des revendication 19 à 21, dans lequel l'organe distributeur est équipé d'un capteur de température (10), par exemple au niveau d'une extrémité libre.

23. Appareil selon l'une des revendication 19 à 22, dans lequel la température de consigne est définie en fonction de la substance.

24. Appareil selon la revendication 23, dans lequel le dispositif de commande est relié à un détecteur configuré pour détecter un marquage au niveau du récipient de stockage (5, 106, 300, 400, 550) indicatif de la substance contenue dans le récipient et dans lequel le dispositif de commande détermine en fonction dudit marquage, au moins un paramètre de fonctionnement de l'appareil parmi la température de consigne, un débit d'air et des indications temporelles définissant un cycle arrêt/marche.

25. Appareil selon l'une des revendication 19 à 24, dans lequel le dispositif de commande (130, 230) comporte une mémoire stockant une table de valeurs associant des substances à des températures de consigne.

26. Appareil selon la revendication 25, comportant en outre un module de communication pour assurer une communication filaire ou non filaire avec un serveur de données, afin de modifier la table de valeurs.

27. Appareil selon l'une des revendications 1 à 26, dans lequel la substance à la deuxième température se répand dans l'état liquide sur une surface de l'organe distributeur située dans le système d'aération.

28. Appareil selon l'une des revendications 1 à 27, dans lequel l'organe de chauffage (11, 132, 211) est configuré pour réguler un débit de la substance à travers l'organe distributeur en modifiant une viscosité de la substance sans vaporiser la substance.

29. Appareil selon l'une des revendications 1 à 28, dans lequel la deuxième température est choisie de manière que l'écoulement de la substance s'effectue à un débit suffisamment faible pour éviter la formation de gouttes se détachant de l'organe distributeur et suffisamment grand pour que la zone d'évaporation reste mouillée en permanence malgré le débit d'air envoyé par le système d'aération.

30. Appareil selon la revendication 29, dans lequel le récipient de stockage (300) ne présente pas d'autre ouverture que l'orifice de vidange, ledit récipient de stockage contenant, outre la substance liquide, une phase gazeuse occupant au moins 20% du volume du récipient de stockage.

31. Appareil selon la revendication 29, dans lequel le récipient de stockage (400) comporte un réservoir extérieur (402) et un réservoir intérieur (403) logé dans le réservoir extérieur, le réservoir intérieur étant en liaison avec l'organe distributeur à travers l'orifice de vidange et présentant un évent (401) relié à l'atmosphère à une extrémité opposée à l'orifice de vidange, un orifice de communication entre le réservoir extérieur et le réservoir intérieur étant agencé à proximité de l'orifice de vidange, le réservoir extérieur ne présentant pas d'autre ouverture que l'orifice de communication.

32. Appareil selon l'une des revendications 1 à 31, dans lequel le récipient de stockage (5, 106, 300, 400, 550) est monté de manière amovible dans l'appareil et configuré de manière à pouvoir être retiré de l'appareil sans perte de substance.

33. Appareil selon la revendication 32, dans lequel le récipient de stockage est monté dans l'appareil par vissage ou encliquetage.

34. Appareil selon l'une des revendications 1 à 33, dans lequel l'organe distributeur présente une première surface tournée vers le récipient de stockage et munie d'un joint d'étanchéité (214) assurant une liaison étanche entre l'organe distributeur et le récipient de stockage, et une deuxième surface agencée dans le système d'aération.

35. Appareil selon l'une des revendications 1 à 34, dans lequel le récipient de stockage (300) comprend un joint d'étanchéité (308) agencé autour de l'orifice de vidange, de manière à assurer une liaison étanche entre le récipient de stockage et l'organe distributeur.

36. Appareil selon l'une des revendications 1 à 35, dans lequel le récipient de stockage (400) comprend un organe de rétention alvéolaire (408) agencée dans le récipient de manière adjacente à l'orifice de vidange pour limiter un écoulement de la substance.

37. Appareil selon l'une des revendications 1 à 36, dans lequel la substance présente une température d'ébullition comprise entre 30°C et 400°C à la pression atmosphérique.

38. Appareil selon l'une des revendications 1 à 37, dans lequel la substance présente une viscosité supérieure à 1 cPa.s à 25°C et inférieure à 1 cPa.s à 60°C.

39. Appareil selon l'une des revendication 1 à 38, dans lequel la substance est une solution comportant au moins un composé pris dans le groupe formé par les agents odorifères utilisables pour l'homme ou l'animal, les substances sémiochimiques, les agents cosmétiques, les huiles essentielles, les parfums et les agents phytosanitaires et agricoles.

40. Appareil selon la revendication 39, dans lequel les agents odorifères utilisables pour l'animal sont choisis parmi les acides gras ou la forme estérifiée desdits acides gras telle que l'oléate de méthyle, le palmitate de méthyle, l'azélate de diméthyle, et le pimelate de diméthyle.

41. Appareil selon la revendication 39, dans lequel la substance est une solution renfermant au moins une substance sémiochimique, au moins une phéromone, une allomone ou une kairomone, d'origine naturelle ou synthétique.

42. Appareil selon la revendication 39, dans lequel la substance est une solution renfermant au moins une phéromone sexuelle ou non, une allomone, une synomone ou une kairomone destinée à provoquer une réponse positive ou négative relativement à l'espèce visée, dont le résultat comportemental peut être une confusion sexuelle, une confusion d'autre nature, une attraction sexuelle, une attraction d'autre nature, une répulsion de toute nature, chez les arthropodes, dont les arachnides, ou dont les hexapodes, parmi lesquels notamment les insectes, dont les insectes nuisibles.

43. Appareil selon la revendication 39, dans lequel la substance est une solution renfermant au moins une phéromone ou une phéromone sexuelle, une allomone, une synomone ou une kairomone destinée à provoquer une réponse positive ou négative relativement à l'espèce visée, dont le résultat comportemental peut être notamment un apaisement, une relaxation, une euphorisation ou une intimidation chez les classes mammalia et aves.

44. Appareil selon l'une des revendications 1 à 43, dans lequel la substance comprend un solvant choisi parmi le myristate d'isopropyle, le dipropylène glycol, Éther de dipropylène glycol monométhylique, et un hydrocarbure isoparaffinique.

45. Appareil selon l'une des revendications 1 à 44, comportant une pluralité de récipients de stockage (5,5a,5b,5c) contenant chacun une substance sous forme liquide ou plusieurs substances sous forme liquide et miscibles entre elles.

46. Appareil selon la revendication 45, dans lequel tout ou partie de l'ensemble des récipients de stockage (5,5a,5b,5c) est porté extérieurement par la canalisation (2) du système d'aération.

47. Appareil selon l'une des revendications 45 à 46, dans lequel chaque récipient de stockage (5) est associé à un corps poreux (8) de l'organe distributeur, l'ensemble des corps poreux (8) étant mis en place à l'intérieur de la canalisation du système d'aération et étant disposés avec des décalages des corps poreux (8), dans une direction longitudinale de la canalisation.

48. Appareil selon l'une des revendications 1 à 47, dans lequel la première température est égale à 30°C.

49. Appareil selon l'une des revendications 1 à 47, dans lequel la première température est égale à 50°C et la substance est une solution de Codlémone.

## Patentansprüche

1. Apparat zum Dispergieren einer flüssigen Substanz im Dampfzustand, welche in einem Speicherbehälter enthalten ist, bei Umgebungstemperatur, wobei der Apparat umfasst:
- ein Belüftungssystem umfassend Rohrleitungen (2, 4, 510), welche in der freien Luft münden und konfiguriert sind, um den Durchlass eines Luftstromes in die Rohrleitungen zu ermöglichen;
- mindestens ein Verteilerorgan (8, 208), welches dazu bestimmt ist, mit einer flüssigen Substanz durch den Speicherbehälter versorgbar zu sein, wobei das Verteilerorgan Mikrorohrleitungen umfasst, welche einen Ausgang, welcher in den Rohrleitungen angeordnet ist, bilden, um dort einen Verdampfungsbereich der Substanz zu bilden,
- mindestens ein Speicherbehälter (5, 106, 300, 400, 550), welcher die Substanz enthält und mit einem Verteilungsorgan (8, 208) verbunden ist, wobei der Behälter eine Entleerungsöffnung umfasst, welche mit dem Verteilerorgan verbunden ist, und
- ein Heizorgan (11, 211, 132), welches auf dem oder in dem Verteilerorgan angeordnet ist, sodass es den Fluss der Substanz durch das Verteilerorgan kontrolliert,
wobei die Entleerungsöffnung nach unten gerichtet ist, wenn der Apparat in einer Betriebsstellung ist, wobei das Verteilerorgan in der Betriebsstellung unter der Entleerungsöffnung angeordnet ist, und dadurch, dass die Substanz eine veränderliche Viskosität in Abhängigkeit von der Temperatur aufweist, wobei der Apparat **dadurch gekennzeichnet ist, dass** die Viskosität derart ist, dass in der Betriebsstellung bei einer Umgebungstemperatur unter einer ersten Temperatur die Substanz nicht durch die Mikrorohrleitungen des Verteilerorgans fließen kann, wobei das Heizorgan konfiguriert ist, um das Verteilerorgan bis zu einer zweiten Temperatur, welche höher ist als die erste Temperatur, zu erhitzen, sodass ein Fluss der Substanz durch die Mikrorohrleitungen des Verteilerorgans in der Betriebsstellung durch die Kapillarität stattfindet.

2. Apparat gemäß Anspruch 1, wobei das Verteilerorgan einen porösen Körper (8, 208), welcher Poren aufweist, umfasst, wobei die Poren mindestens einen Teil der Mikrorohrleitungen des Verteilerorgans bilden.

3. Apparat gemäß Anspruch 2, wobei die Poren einen Durchmesser zwischen 0.01 und 10 µm aufweisen.

4. Apparat gemäß einem der Ansprüche 2 bis 3, wobei der poröse Körper (8, 208) eine zylindrische Form aufweist.

5. Apparat gemäß Anspruch 4, wobei die Substanzzufuhr in einer Blindaussparung (9), welche parallel zur Achse des porösen Körpers (8) vorgesehen ist, aufnehmbar ist.

6. Apparat gemäß Anspruch 4, wobei der poröse Körper einen Vorsprung (208b) umfasst, welcher auf einem oberen Teil des porösen Körpers angeordnet ist und entsprechend einer Längsachse des porösen Körpers verläuft, und konfiguriert ist, um die Substanz aufzunehmen.

7. Apparat gemäß einem der Ansprüche 2 bis 6, wobei das Verteilerorgan (8) eine umlaufende Membran umfasste, welche um den porösen Körper angeordnet ist und Löcher aufweist, welche die Mikrorohrleitungen bilden.

8. Apparat gemäß einem der Ansprüche 2 bis 7, wobei der poröse Körper (8, 208) eine Porosität in einem unteren Teil des porösen Körpers aufweist, welche schwächer ist, als eine Porosität in einem äußeren Bereich des porösen Körpers, welcher den inneren Bereich umgibt.

9. Apparat gemäß einem der Ansprüche 2 bis 8, wobei der poröse Körper einen Docht aus Holz, aus Stoff, aus Keramik oder aus Polymer umfasst.

10. Apparat gemäß einem der Ansprüche 2 bis 9, wobei das Heizorgan (11, 132, 211) unmittelbar auf einer Fläche des porösen Körpers angeordnet ist.

11. Apparat gemäß einem der Ansprüche 2 bis 9, wobei der poröse Körper mindestens eine Aussparung (210) umfasst, welche mindestens einen Teil des Heizorganes aufnimmt.

12. Apparat gemäß einem der Ansprüche 1 bis 11, wobei das Verteilerorgan eine Hohlnadel (220) umfasst, welche konfiguriert ist, um einen Deckel zu durchbrechen und/oder eine Membran (310), welche die Klappe des Speicherbehälters bildet, zu bewegen und die Substanz, welche in dem Speicherbehälter enthalten ist, bis zur Verdampfungsfläche zu führen.

13. Apparat gemäß Anspruch 12 in Verbindung mit einem der Ansprüche 2 bis 11, wobei die Nadel (220) in einem der Endbereiche des porösen Körpers angeordnet ist.

14. Apparat gemäß einem der Ansprüche 1 bis 13, wobei die Mikrorohrleitungen einen Querschnitt zwischen 10⁻⁴ µm² und 10⁶ µm², vorzugsweise zwischen 0,1 µm² und103 µm², aufweisen.

15. Apparat gemäß einem der Ansprüche 1 bis 14, umfassend weiterhin ein Befestigungsorgan, welches in Richtung und/oder in Neigung zu den Rohrleitungen des Belüftungssystems ausrichtbar ist, um die Rohrleitungen im Verhältnis zum Boden auszurichten, wenn das Befestigungsorgan auf einem Träger befestigt ist.

16. Apparat gemäß einem der Ansprüche 1 bis 15, wobei das Belüftungssystem mindestens ein Ventilator (1), welcher in einem Bereich der Rohrleitungen (2, 4, 510) angeordnet ist, umfasst.

17. Apparat gemäß einem der Ansprüche 1 bis 16, wobei das Belüftungssystem Öffnungen umfasst, welche in einer Stirnwand der Rohrleitungen (2, 4) ausgebildet sind, und verstellbare Verschlüsse, welche die Öffnungen ausstatten, um einen Durchflussquerschnitt der Öffnungen zu regulieren.

18. Apparat gemäß einem der Ansprüche 16 oder 17, wobei der Apparat ein Steuerorgan (130, 530) eines Luftstroms in den Rohrleitungen umfasst, konfiguriert, um den Ventilator und/oder die Verschlüsse zu kontrollieren, um den Luftstrom in den Rohrleitungen zu regulieren.

19. Apparat gemäß einem der Ansprüche 1 bis 18, umfassend weiterhin eine Steuervorrichtung (130, 530), konfiguriert, um das Heizorgan (11, 132, 211) in Abhängigkeit einer Solltemperatur im Verteilerorgan zu steuern.

20. Apparat gemäß Anspruch 19, wobei das Heizorgan mindestens eine elektrische Karte (130, 230) und mindestens einen elektrischen Widerstand (211), welcher durch die elektrische Karte versorgt ist, umfasst.

21. Apparat gemäß Anspruch 20, wobei die Steuervorrichtung auf der elektrischen Karte (130) angeordnet ist.

22. Apparat gemäß einem der Ansprüche 19 bis 21, wobei das Verteilerorgan mit einem Temperatursensor (10) ausgestattet ist, z.B. auf Höhe eines freien Endabschnittes.

23. Apparat gemäß einem der Ansprüche 19 bis 22, wobei der Temperatursensor in Abhängigkeit der Substanz definiert ist.

24. Apparat gemäß Anspruch 23, wobei die Steuervorrichtung mit einem Detektor verbunden ist, welcher konfiguriert ist, um eine Markierung im Bereich des Speicherbehälters (5, 106, 300, 400, 550), welche die Substanz in dem Behälter anzeigt, zu erkennen, und wobei die Steuervorrichtung in Abhängigkeit der Markierung mindestens ein Betriebsparameter des Apparates ausgewählt zwischen der Solltemperatur, des Luftstromes und temporären Indikationen, welche einen Aus/Ein- Zyklus definieren, bestimmt.

25. Apparat gemäß einem der Ansprüche 19 bis 24, wobei die Steuervorrichtung (130, 230) einen Speicher umfasst, welcher eine Wertetabelle speichert, die die Substanzen mit den Solltemperaturen verbindet.

26. Apparat gemäß Anspruch 25, umfassend weiterhin ein Kommunikationsmodul, um eine drahtgebundene oder drahtlose Kommunikation mit einem Datenserver zu gewährleisten, um die Wertetabelle zu verändern.

27. Apparat gemäß einem der Ansprüche 1 bis 26, wobei die Substanz mit der zweiten Temperatur sich in einem flüssigen Zustand auf einer Fläche des Verteilerorgans, welches in dem Belüftungssystem angeordnet ist, verteilt.

28. Apparat gemäß einem der Ansprüche 1 bis 27, wobei das Heizorgan (11, 132, 211) konfiguriert ist, um einen Durchfluss der Substanz durch das Verteilerorgan durch Änderung der Viskosität der Substanz zu regeln, ohne die Substanz zu verdampfen.

29. Apparat gemäß einem der Ansprüche 1 bis 28, wobei die zweite Temperatur so ausgesucht ist, dass der Strom der Substanz mit einem so geringen Durchfluss stattfindet, um die Bildung von Tropfen, welche sich vom Verteilerorgan lösen, zu verhindern und mit einem ausreichend großen Durchfluss, damit der Verdampfungsbereich trotz des durch das Belüftungssystem übermittelten Luftstromes durchgehend befeuchtet bleibt.

30. Apparat gemäß Anspruch 29, wobei der Speicherbehälter (300) keine andere Öffnung als die Entleerungsöffnung aufweist, wobei der Speicherbehälter neben der flüssigen Substanz eine Gasphase enthält, welche mindestens 20% des Volumens des Speicherbehälters einnimmt.

31. Apparat gemäß Anspruch 29, wobei der Speicherbehälter (400) einen Außenbehälter (402) und einen Innenbehälter (403), welcher in dem Außenbehälter angeordnet ist, umfasst, wobei der Innenbehälter durch die Entleerungsöffnung in Verbindung mit dem Verteilerorgan steht und eine Entlüftung (401) aufweist, welche an einem der Entleerungsöffnung gegenüberliegenden Endabschnitt mit der Atmosphäre verbunden ist, wobei eine Verbindungsöffnung zwischen dem Außenbehälter und dem Innenbehälter in der Nähe der Entleerungsöffnung angebracht ist, wobei der Außenbehälter keine andere Öffnung als die Verbindungsöffnung aufweist.

32. Apparat gemäß einem der Ansprüche 1 bis 31, wobei der Speicherbehälter (5, 106, 300, 400, 550) herausnehmbar in dem Apparat angebracht ist und so konfiguriert ist, dass er aus dem Apparat ohne Substanzverlust entnommen werden kann.

33. Apparat gemäß Anspruch 32, wobei der Speicherbehälter in dem Apparat durch Verschraubung oder Verrastung angebracht ist.

34. Apparat gemäß einem der Ansprüche 1 bis 33, wobei das Verteilerorgan eine erste Fläche aufweist, die hin zum Speicherbehälter gerichtet ist und mit einer Dichtung (214) ausgestattet ist, so dass eine dichte Verbindung zwischen dem Verteilerorgan und dem Speicherbehälter gewährleistet ist, und eine zweite Fläche, welche in dem Belüftungssystem angeordnet ist.

35. Apparat gemäß einem der Ansprüche 1 bis 34, wobei der Speicherbehälter (300) eine Dichtung (308) umfasst, welche um die Entleerungsöffnung angeordnet ist, so dass eine dichte Verbindung zwischen dem Speicherbehälter und dem Verteilerorgan gewährleistet ist.

36. Apparat gemäß einem der Ansprüche 1 bis 35, wobei der Speicherbehälter (400) ein zellenförmiges Rückhalteorgan (408), welches in dem Behälter angrenzend zur Entleerungsöffnung angeordnet ist, um den Substanzfluss einzugrenzen, umfasst.

37. Apparat gemäß einem der Ansprüche 1 bis 36, wobei die Substanz eine Siedetemperatur zwischen 30°C und 400°C bei Atmosphärendruck aufweist.

38. Apparat gemäß einem der Ansprüche 1 bis 37, wobei die Substanz bei 25°C eine Viskosität über 1 cPa.s und bei 60°C eine Viskosität unter1 cPa.s aufweist.

39. Apparat gemäß einem der Ansprüche 1 bis 38, wobei die Substanz eine Lösung ist, welche mindestens einen Bestandteil ausgesucht aus der Gruppe bestehend aus duftenden Mitteln verwendbar für Mensch oder Tier, semiochemischen Substanzen, kosmetische Mittel, ätherischen Ölen, Parfüms und Pflanzenschutz- oder Agrarmittel, enthält.

40. Apparat gemäß Anspruch 39, wobei die duftenden Mittel verwendbar für Tiere ausgesucht sind zwischen Fettsäuren oder veresterten Formen dieser Fettsäuren, wie z.B. Methyloleat, Methylpalmitat, Dimethylazelat und Dimethylprimelat.

41. Apparat gemäß Anspruch 39, wobei die Substanz eine Lösung ist umfassend mindestens eine semiochemische Lösung, mindestens ein Pheromon, ein Allomon oder ein Kairomon, natütlicher oder synthetischer Art.

42. Apparat gemäß Anspruch 39, wobei die Substanz eine Lösung ist welche mindestens einschließt ein sexuelles oder nicht sexuelles Pheromon, ein Allomon, ein Synomon oder ein Kairomon, welche dazu bestimmt sind, eine positive oder negative Antwort im Verhältnis zur Zielart zu erhalten, deren Verhaltensergebnis eine sexuelle Verwirrung, eine Verwirrung anderer Natur, eine sexuelle Anziehung, eine Anziehung anderer Natur, eine Zurückweisung jeglicher Natur, bei den Gliederfüßler, darunter Spinnentiere oder Sechsfüßler, darunter insbesondere Insekten, darunter Schädlinge auszulösen.

43. Apparat gemäß Anspruch 39, wobei die Substanz ein Mittel ist, welches mindestens einschließt ein Pheromon oder ein sexuelles Pheromon, ein Allomon, ein Synomon oder ein Kairomon, welche dazu bestimmt sind eine positive oder negative Antwort im Verhältnis zur Zielart zu erhalten, deren Verhaltensergebnis insbesondere sein kann eine Beruhigung, eine Entspannung, eine Euphorisierung oder eine Einschüchterung bei der Spezies Mammalia und Aves.

44. Apparat gemäß einem der Ansprüche 1 bis 43, wobei die Substanz ein Lösungsmittel enthält, welches ausgesucht wird aus dem Isopropylmyristat, dem Dipropylenglykol, dem Dipropylenglycolmonomethylether und einem isoporaffinischer Kohlenwasserstoff.

45. Apparat gemäß einem der Ansprüche 1 bis 44, umfassend eine Vielzahl von Speicherbehältern (5, 5a, 5b, 5c), welche jeder eine flüssige Substanz oder mehrere flüssige Substanzen, welche untereinander mischbar sind, enthalten.

46. Apparat gemäß Anspruch 45, wobei das Gesamte oder ein Teil der Einheit der Speicherbehälter (5, 5a, 5b, 5c) Außen getragen ist durch die Rohrleitungen (2) des Belüftungssystemes.

47. Apparat gemäß einem der Ansprüche 45 bis 46, wobei jeder Speicherbehälter (5) mit einem porösen Körper (8) des Verteilerorgans verbunden ist, wobei die Gesamtheit der porösen Körper (8) im Inneren der Rohrleitungen des Belüftungssystemes errichtet sind und die porösen Körper versetzt angeordnet sind.

48. Apparat gemäß einem der Ansprüche 1 bis 47, indem die erste Temperatur 30°C ist.

49. Apparat gemäß einem der Ansprüche 1 bis 47, wobei die erste Temperatur 50°C ist und die Substanz eine Codlemon-Lösung ist.

## Claims

1. An apparatus for dispersing in the air, in the vapor state, a substance which is in the liquid state at ambient temperature, and is contained in a storage container, the apparatus comprising:
- a ventilation system comprising a duct (2, 4, 510) which opens out into the open air, and is configured to permit the passage of a flow of air into the duct;
- at least one dispensing unit (8, 208) which is designed to be supplied with a liquid substance by the storage container, the dispensing unit comprising micro-ducts forming an outlet which is provided in the duct in order to constitute an area of evaporation of the substance therein;
- at least one storage container (5, 106, 300, 400, 550) which contains the substance and is connected to the dispensing unit (8, 208), wherein the storage container (5, 300, 400, 550) has a discharge orifice which is connected to the dispensing unit, and
- a heating unit (11, 211, 132) which is provided on or in the dispensing unit, such as to control a flow of the substance through the dispensing unit, the discharge orifice is oriented downwards when the apparatus is in a position of use, the dispensing unit being below the discharge orifice in the position of use, and in that the substance has a viscosity which is variable according to the temperature, the apparatus being **characterized in that** said viscosity is such that the substance can not flow through the micro-ducts of the dispensing unit at an ambient temperature lower than a first temperature, the heating unit being configured to heat the dispensing unit to a second temperature higher than the first temperature, such that a flow of the substance through the micro-ducts of the dispensing unit takes place by capillarity in the position of use.

2. The apparatus as claimed in claim 1, wherein the dispensing unit comprises a porous body (8, 208) comprising pores, said pores constituting at least part of the micro-ducts of the dispensing unit.

3. The apparatus as claimed in claim 2, wherein the pores have a diameter contained between 0.01 and 10 µm.

4. The apparatus as claimed in one of claims 2 to 3, wherein the porous body (8, 208) is in the form of a cylinder.

5. The apparatus as claimed in claim 4, wherein the supply of substance is received in a blind recess (9) provided parallel to the axis of the porous body (8).

6. The apparatus as claimed in claim 4, wherein the porous body comprises a lug (208b) which is provided on an upper part of said body, extends along a longitudinal axis of the porous body, and is configured to receive the substance.

7. The apparatus as claimed in one of claims 2 to 6, wherein the dispensing unit (8) comprises a peripheral membrane which is provided around the porous body, and is pierced with holes constituting micro-ducts.

8. The apparatus as claimed in one of claims 2 to 7, wherein the porous body (8, 208) has porosity in an inner part of the porous body which is lower than porosity in an outer part of the porous body surrounding the inner part.

9. The apparatus as claimed in one of claims 2 to 8, wherein the porous body comprises a wick which is made of wood, textile, ceramic or polymer.

10. The apparatus as claimed in one of claims 2 to 9, wherein the heating unit (11, 132, 211) is placed directly on a surface of the porous body.

11. The apparatus as claimed in one of claims 2 to 9, wherein the porous body has at least one recess (210) which accommodates at least part of the heating unit.

12. The apparatus as claimed in one of claims 1 to 11, wherein the dispensing unit comprises a hollow needle (220) which is configured to pierce a cap and/or to displace a membrane (310) which acts as a shutter of the storage container, and bring the substance contained in the storage container to the evaporation surface.

13. The apparatus as claimed in claim 12 taken in combination with one of claims 2 to 11, wherein the needle (220) is disposed at one of the ends of the porous body.

14. The apparatus as claimed in one of claims 1 to 13, wherein the micro-ducts have a cross-section contained between 10⁻⁴ µm² and 10⁶ µm², preferably between entre 0.1 µm² and 10³ µm².

15. The apparatus as claimed in one of claims 1 to 14, additionally comprising a securing unit, the direction and/or inclination of which can be oriented relative to the duct of the ventilation system, in order to orient the duct relative to the ground when the securing unit is secured on a support.

16. The apparatus as claimed in one of claims 1 to 15, wherein the ventilation system comprises at least one fan (1) which is placed in part of the duct (2, 4, 510).

17. The apparatus as claimed in one of claims 1 to 16, wherein the ventilation system comprises openings provided in an end wall of the duct (2, 4), and adjustable shutters which equip said openings, in order to make it possible to regulate a cross-section of passage of the openings.

18. The apparatus as claimed in claim 16 or 17, wherein said apparatus comprises a regulator unit (130, 530) for a flow of air in the duct, which unit is configured to control the fan and/or the shutters, in order to regulate a flow of air in the duct.

19. The apparatus as claimed in one of claims 1 to 18, additionally comprising a control device (130, 530) which is configured to control the heating unit (11, 132, 211) according to a set temperature in the dispensing unit.

20. The apparatus as claimed in claim 19, wherein the heating unit comprises at least one electronic board (130, 230) and at least one electrical resistor (211) which is supplied electrically by the electronic board.

21. The apparatus as claimed in claim 20, wherein the control device is provided on the electronic board (130).

22. The apparatus as claimed in one of claims 19 to 21, wherein the dispensing unit is equipped with a temperature sensor (10), for example at a free end.

23. The apparatus as claimed in one of claims 19 to 22, wherein the set temperature is defined according to the substance.

24. The apparatus as claimed in claim 23, wherein the control device is connected to a detector which is configured to detect marking at the storage container (5, 106, 300, 400, 550) indicating the substance contained in the container, and wherein according to said marking the control device determines at least one operating parameter of the apparatus from out of the set temperature, a flow of air, and temporal indications defining a stop/operating cycle.

25. The apparatus as claimed in one of claims 19 to 24, wherein the control device (130, 230) comprises a memory which stores a table of values associating substances with set temperatures.

26. The apparatus as claimed in claim 25, additionally comprising a communication module, in order to ensure wired or non-wired communication with a data server, in order to modify the table of values.

27. The apparatus as claimed in one of claims 1 to 26, wherein the substance at the second temperature spreads in the liquid state on a surface of the dispensing unit, which surface is situated in the ventilation system.

28. The apparatus as claimed in one of claims 1 to 27, wherein the heating unit (11, 132, 211) is configured to regulate a flow of the substance through the dispensing unit by modifying a viscosity of the substance without vaporizing the substance.

29. The apparatus as claimed in one of claims 1 to 28, wherein the second temperature is selected such that the flow of the substance takes place at a flow rate which is sufficiently low to prevent the formation of drops becoming detached from the dispensing unit, and sufficiently great for the evaporation area to remain permanently wetted despite the flow of air sent by the ventilation system.

30. The apparatus as claimed in claim 29, wherein the storage container (300) does not have an opening other than the discharge orifice, said storage container containing a gaseous phase which occupies at least 20% of the volume of the storage container, as well as the liquid substance.

31. The apparatus as claimed in claim 29, wherein the storage container (400) comprises an outer tank (402) and an inner tank (403) which is accommodated in the outer tank, the inner tank being connected to the dispensing unit via the discharge orifice, and having a vent (401) which is connected to the atmosphere at an end opposite the discharge orifice, an orifice for communication between the outer tank and the inner tank being provided in the vicinity of the discharge orifice, the outer tank not having an opening other than the communication orifice.

32. The apparatus as claimed in one of claims 1 to 31, wherein the storage container (5, 106, 300, 400, 550) is fitted removably in the apparatus, and is configured such as to be able to be removed from the apparatus without loss of substance.

33. The apparatus as claimed in claim 32, wherein the storage container is fitted in the apparatus by being screwed or snapped in.

34. The apparatus as claimed in one of claims 1 to 33, wherein the dispensing unit has a first surface which faces towards the storage container, and is provided with a seal (214) ensuring a sealed connection between the dispensing unit and the storage container, and a second surface which is provided in the ventilation system.

35. The apparatus as claimed in one of claims 1 to 34, wherein the storage container (300) comprises a seal (308) which is provided around the discharge orifice such as to ensure a sealed connection between the storage container and the dispensing unit.

36. The apparatus as claimed in one of claims 1 to 35, wherein the storage container (400) comprises an alveolar retention unit (408) which is provided in the container in a manner adjacent to the discharge orifice, in order to limit a flow of the substance.

37. The apparatus as claimed in one of claims 1 to 36, wherein the substance has a boiling temperature contained between 30°C and 400°C at atmospheric pressure.

38. The apparatus as claimed in one of claims 1 to 37, wherein the substance has a viscosity greater than 1 cPa.s at 25°C, and less than 1 cPa.s at 60°C.

39. The apparatus as claimed in one of claims 1 to 38, wherein the substance is a solution comprising at least one compound taken from the group formed by odiferous agents which can be used for people or animals, semiochemical substances, cosmetic agents, essential oils, perfumes and phytosanitary and agricultural agents.

40. The apparatus as claimed in claim 39, wherein the odiferous agents which can be used for animals are selected from amongst fatty acids or the esterified form of said fatty acids, such as methyl oleate, methyl palmitate, dimethyl azelate, and dimethyl pimelate.

41. The apparatus as claimed in claim 39, wherein the substance is a solution which contains at least one semiochemical substance, at least one pheromone, an allomone or a kairomone of natural or synthetic origin.

42. The apparatus as claimed in claim 39, wherein the substance is a solution containing at least one pheromone which is or is not sexual, an allomone, a synomone or a kairomone, designed to provoke a positive or negative response relative to the species concerned, the behavioral result of which can be sexual confusion, confusion of another kind, sexual attraction, attraction of another kind, repulsion of any kind, in arthropods, including arachnids, or including hexapods, which includes in particular insects, including harmful insects.

43. The apparatus as claimed in claim 39, wherein the substance is a solution containing at least one pheromone or a sexual pheromone, an allomone, a synomone or a kairomone designed to provoke a positive or negative response relative to the species concerned, the behavioral result of which can in particular be pacification, relaxation, mood elevation, or intimidation, in the classes mammalia and aves.

44. The apparatus as claimed in one of claims 1 to 43, wherein the substance comprises a solvent selected from amongst isopropyl myristate, dipropylene glycol, monomethyl dipropylene glycol ether, and an isoparaffinic hydrocarbon.

45. The apparatus as claimed in one of claims 1 to 44, comprising a plurality of storage containers (5, 5a, 5b, 5c) each containing a substance in liquid form, or a plurality of substances in liquid form which are miscible with one another.

46. The apparatus as claimed in claim 45, wherein all or part of the assembly of the storage containers (5, 5a, 5b, 5c) is supported on the exterior by the duct (2) of the ventilation system.

47. The apparatus as claimed in one of claims 45 to 46, wherein each storage container (5) is associated with a porous body (8) of the dispensing unit, the assembly of the porous bodies (8) being placed in the interior of the duct of the ventilation system, and being disposed with offsettings of the porous bodies (8) in a longitudinal direction of the duct.

48. The apparatus as claimed in one of claims 1 to 47, wherein the first temperature is equal to 30°C.

49. The apparatus as claimed in one of claims 1 to 47, wherein the first temperature is equal to 50°C and the substance is a solution of Codlémone.
